(19) Europäisches Patentamt
European Patent Office
Office européen des brevets

(11) Veröffentlichungsnummer: **0 240 899 B1**

(12) **EUROPÄISCHE PATENTSCHRIFT**

(45) Veröffentlichungstag der Patentschrift: **19.08.92**

(21) Anmeldenummer: **87104717.1**

(22) Anmeldetag: **31.03.87**

(51) Int. Cl.[5]: **C07D 487/04**, C07D 495/14, A61K 31/55, //(C07D487/04, 243:00,235:00),(C07D487/04, 249:00,243:00),(C07D495/14, 333:00,249:00,243:00)

# (54) 1,4-Diazepine.

(30) Priorität: **01.04.86 DE 3610848**

(43) Veröffentlichungstag der Anmeldung:
**14.10.87 Patentblatt 87/42**

(45) Bekanntmachung des Hinweises auf die Patenterteilung:
**19.08.92 Patentblatt 92/34**

(84) Benannte Vertragsstaaten:
**AT BE CH DE ES FR GB GR IT LI LU NL SE**

(56) Entgegenhaltungen:
**EP-A- 0 033 974**
**DE-A- 2 321 705**
**DE-A- 2 456 311**
**US-A- 3 928 589**

(73) Patentinhaber: **BOEHRINGER INGELHEIM KG**
**Postfach 200**
**W-6507 Ingelheim am Rhein(DE)**
(84) Benannte Vertragsstaaten:
**BE CH DE ES FR GR IT LI LU NL SE AT**

(73) Patentinhaber: **BOEHRINGER INGELHEIM INTERNATIONAL GmbH**
**Postfach 200**
**W-6507 Ingelheim am Rhein(DE)**
(84) Benannte Vertragsstaaten:
**GB**

(72) Erfinder: **Harreus, Albrecht, Dr.**
**Sandstrasse 1**
**W-6507 Ingelheim am Rhein(DE)**
Erfinder: **Weber, Karl-Heinz, Dr.**
**Kaiser-Karl-Strasse 11**
**W-6535 Gau-Algesheim(DE)**
Erfinder: **Stransky, Werner, Dr.**
**Im Hippel 24**
**W-6535 Gau-Algesheim(DE)**
Erfinder: **Walther, Gerhard, Dr.**
**Pfarrer-Heberer-Strasse 37**
**W-6530 Bingen(DE)**

EP 0 240 899 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Erfinder: **Muacevic, Gojko, Dr.**
**In der Dörrwiese 13**
**W-6507 Ingelheim am Rhein(DE)**
Erfinder: **Casals Stenzel, Jorge, Dr.**
**Hirsch-Gereuth-Strasse 76**
**W-8000 München 70(DE)**
Erfinder: **Bechtel, Wolf-Dietrich, Dr.**
**Mühlstrasse 3**
**W-6531 Appenheim(DE)**

## Beschreibung

Die Erfindung betrifft neue 1,4-Diazepine der allgemeinen Formel

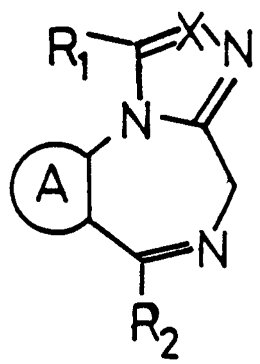

I

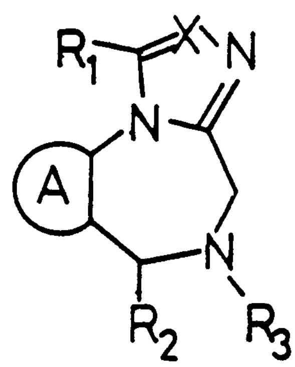

II

worin

- $R_1$ Wasserstoff, eine verzweigte oder unverzweigte Alkylgruppe mit 1 bis 4 Kohlenstoffatomen, die gegebenenfalls durch Hydroxy oder Halogen substituiert sein kann, eine Cyclopropylgruppe, eine Alkoxygruppe mit 1 bis 4 Kohlenstoffatomen, bevorzugt Methoxy, Halogen bevorzugt Chlor oder Brom;
- $R_2$ Phenyl, wobei der Phenylring, bevorzugt in 2-Stellung, ein- oder mehrfach durch Methyl, Methoxy, Halogen, bevorzugt Chlor oder Brom, Nitro und/oder Trifluormethyl substituiert sein kann, $\alpha$-Pyridyl;
- $R_3$ Wasserstoff oder eine verzweigte oder unverzweigte Alkylgruppe mit 1 bis 4 Kohlenstoffatomen, bevorzugt Methyl;
- A einen ankondensierten Ring der allgemeinen Formel

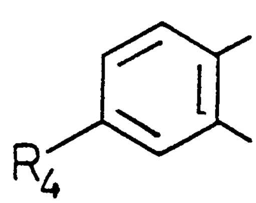

a

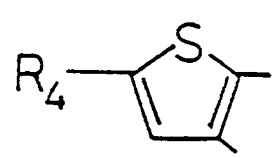

b

worin $R_4$ eine funktionelle Seitenkette der allgemeinen Formel

$$-Y_n-\overset{O}{\overset{||}{C}}-\langle AS\rangle_m-R_5$$

worin Y eine verzweigte oder unverzweigte Alkylgruppe mit n Kohlenstoffatomen, <AS> eine N-terminal angeknüpfte Aminosäure der allgemeinen Formel

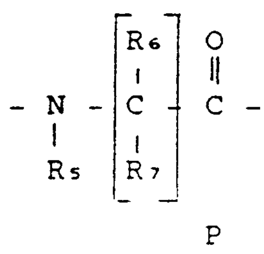

worin

$R_5$ Wasserstoff, eine verzweigte oder unverzweigte Alkylgruppe mit 1 bis 5 Kohlenstoffatomen;

$R_6$ und $R_7$, die gleich oder verschieden sein können, Wasserstoff, eine verzweigte oder unverzweigte Alkyl-, Alkenyl- oder Alkinylgruppe mit 1 bis 10 Kohlenstoffatomen, eine Cycloalkylgruppe mit 3 bis 6 Kohlenstoffatomen, eine Phenylgruppe, eine Benzylgruppe, wobei $R_6$ und/oder $R_7$ im Fall einer $C_1$-$C_4$ Alkylgruppe gegebenenfalls ein- oder mehrfach substituiert ist durch Hydroxy, $C_1$-$C_4$ Alkoxy, Cycloalkyl mit 3 bis 7 Kohlenstoffatomen, Halogen, eine Aminogruppe, die gegebenenfalls ein- oder zweifach durch verzweigtes oder unverzweigtes Alkyl, Alkoxycarbonyl oder Phenalkoxycarbonyl mit jeweils 1 bis 4 Kohlenstoffatomen in der Alkylkette substituiert sein kann, Guanidino, Ureido, Carboxy, $C_1$-$C_4$-Alkoxycarbonyl, Cyano, Carboxamid, $C_1$-$C_4$-Alkylcarbonyl, Mercapto, $C_1$-$C_4$ Alkylmercapto, Benzylmercapto, $C_1$-$C_4$-Alkylsulfoxyl, $C_1$-$C_4$ Alkylsulfonyl, 3-Indolyl, Imidazolyl, Pyrazolyl und/oder ein Säureamid der allgemeinen Formel

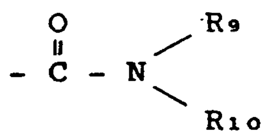

worin $R_9$ und $R_{10}$, die gleich oder verschieden sein können, Wasserstoff, eine verzweigte oder unverzweigte Alkyl-, Alkenyl- oder Alkinylgruppe mit 1 bis 4 Kohlenstoffatomen, wobei die Alkylgruppe durch eine Aminogruppe, die gegebenenfalls ein- oder zweifach durch verzweigtes oder unverzweigtes Alkyl, mit 1 bis 4 Kohlenstoffatomen in der Alkylkette oder durch eine Hydroxygruppe substituiert sein kann

oder

$R_9$ und $R_{10}$ gegebenenfalls einen 3- bis 6-gliedrigen Ring bilden, der als weitere Heteroatome Stickstoff, Sauerstoff oder Schwefel enthalten kann, wobei der Ring durch wenigstens eine verzweigte oder unverzweigte Alkylgruppe mit 1 bis 4 Kohlenstoffatomen, bevorzugt Methyl, substituiert sein kann;

$R_6$ und/oder $R_7$ im Fall einer Phenyl oder einer Benzylgruppe, durch wenigstens eine Hydroxy-, Halogen-, Amino-, verzweigte- oder unverzweigte Alkylamino-, Dialkylamino- oder Alkoxygruppe mit 1 bis 4 Kohlenstoffatomen substituiert sein kann;

$R_8$ eine, gegebenenfalls durch eine Aminogruppe der allgemeinen Formel

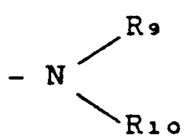

substituierte, verzweigte oder unverzweigte Alkoxy-oder Alkylthiogruppe mit 1 bis 4 Kohlenstoffatomen,

wobei $R_9$ und $R_{10}$ die zuvor genannte Bedeutung aufweisen,

oder eine Aminogruppe der allgemeinen Formel

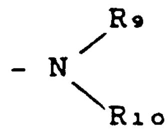

worin $R_9$ und $R_{10}$ die oben genannte Bedeutung haben;

$R_6$ und $R_7$ gegebenenfalls einen 3- bis 7-gliedrigen Ring bilden, der als weitere Heteroatome Stickstoff, Sauerstoff oder Schwefel enthalten kann und gegebenenfalls durch wenigstens eine verzweigte oder unverzweigte Alkylgruppe mit 1 bis 4 Kohlenstoffatomen, bevorzugt Methyl, substituiert sein kann;

oder

$R_5$ und $R_6$ oder $R_7$ gegebenenfalls einen 4- bis 7-gliedrigen Ring bilden, der gegebenenfalls als weitere Heteroatome Stickstoff, Sauerstoff oder Schwefel enthalten kann, wobei der Ring durch wenigstens eine verzweigte oder unverzweigte Alkylgruppe mit 1 bis 4 Kohlenstoffatomen substituiert sein kann;

oder

$R_6$ oder $R_7$ und $R_8$ gegebenenfalls einen 5- bis 7-gliedrigen Ring der allgemeinen Formel

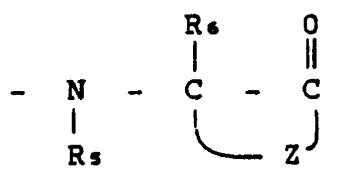

bilden,

wobei

Z eines der Heteroatome Stickstoff, Sauerstoff oder Schwefel bedeutet, wobei der Ring durch eine verzweigte oder unverzweigte Alkylgruppe mit 1 - 4 Kohlenstoffatomen, bevorzugt Methyl, substituiert sein kann;

X CH, C-Halogen oder Stickstoff,

n eine der Zahlen 0, 1, 2, 3, 4, 5, 6, 7 oder 8,

p eine der Zahlen 1, 2, 3, 4, 5, 6, 7 oder 8 und

m eine der Zahlen 1, 2 oder 3, bevorzugt 1, bedeuten, deren optisch aktive Verbindungen oder deren Racemate sowie gegebenenfalls deren physiologisch unbedenkliche Säureadditionssalze.

Bevorzugt sind Verbindungen der allgemeinen Formel I oder II,

worin

$R_1$, $R_3$ und A die zuvor genannte Bedeutung aufweisen,

$R_2$ Phenyl, wobei der Phenylring, bevorzugt in 2-Stellung, durch Halogen, besonders bevorzugt Chlor, substituiert sein kann,

$R_4$ eine funktionelle Seitenkette der allgemeinen Formel

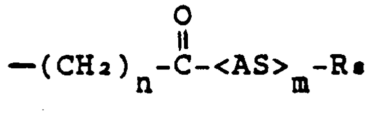

worin

<AS> eine Aminosäure der allgemeinen Formel

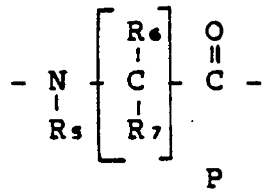

worin

| | |
|---|---|
| $R_5$, $R_6$ und $R_7$ | wie zuvor definiert sind, |
| $R_8$ | die oben angegebene Bedeutung aufweist, |
| X | CH oder Stickstoff, |
| n | eine der Zahlen 0, 1, 2, 3, 4, 5, 6, 7 oder 8 |
| p | die Zahlen 1 oder 2 und |
| m | die Zahlen 1, 2 oder 3, bevorzugt 1, bedeuten, |

deren optisch aktive Verbindungen oder deren Racemate sowie gegebenenfalls deren physiologisch unbedenkliche Säureadditionssalze.

Besonders bevorzugt sind Verbindungen der allgemeinen Formel I oder II,

worin

$R_1$ Wasserstoff, Methyl, Ethyl, Cyclopropyl, Methoxy, Ethoxy oder Halogen, bevorzugt Chlor oder Brom,

$R_2$ Phenyl, wobei der Phenylring in 2-Stellung durch Halogen, bevorzugt Chlor, substituiert sein kann,

$R_3$ Wasserstoff oder Methyl, bevorzugt Wasserstoff,

A wie oben angegeben,

$R_4$ eine funktionell Seitenkette der allgemeinen Formel

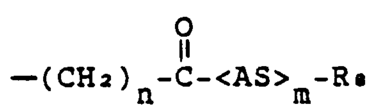

worin

<AS>eine Aminosäure der allgemeinen Formel

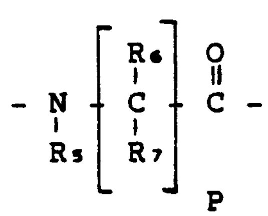

worin

$R_5$, $R_6$ und $R_7$ wie zuvor definiert sind,

$R_8$ wie oben angegeben ist,

X CH oder Stickstoff,

n im Fall von A = a eine der Zahlen 0 oder 1 und im Fall von A = b eine der Zahlen 0, 1, 2, 3, 4, 5, 6, 7 oder 8,

p eine der Zahlen 1 oder 2 und

m eine der Zahlen 1 oder 2, bevorzugt 1,

bedeuten, deren optisch aktive Verbindungen oder deren Racemate sowie deren physiologisch unbedenklichen Säureadditionssalze.

Ganz besonders bevorzugt sind Verbindungen der allgemeinen Formel I oder II, worin

$R_1$ Wasserstoff, Ethyl, Cyclopropyl, Ethoxy, Chlor oder Brom, bevorzugt Methyl oder Methoxy,

$R_2$ Phenyl, wobei der Phenylring in 2-Stellung durch Halogen, bevorzugt Chlor, substituiert sein kann,

$R_3$ Wasserstoff,
A wie oben angegeben,
$R_4$ eine funktionelle Seitenkette der allgemeinen Formel

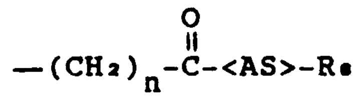

worin
<AS>eine α-Aminosäure der allgemeinen Formel

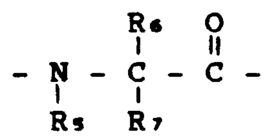

worin
$R_5$ Wasserstoff,
$R_6$ und $R_7$, die gleich oder verschieden sein können, Wasserstoff, eine verzweigte oder unverzweigte Alkylgruppe mit 1 bis 8, bevorzugt 1 bis 5 Kohlenstoffatomen, gegebenenfalls substituiert durch Alkoxycarbonyl, Alkylthio oder Alkylsulfonyl mit 1 bis 4 Kohlenstoffatomen in der Alkylkette,
$R_8$ Alkoxy mit 1 bis 4 Kohlenstoffatomen, bevorzugt Methoxy, Ethoxy oder bevorzugt eine Aminogruppe der allgemeinen Formel

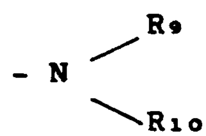

worin
$R_9$ und $R_{10}$, die gleich oder verschieden sein können, eine verzweigte oder unverzweigte Alkyl- oder Alkenylgruppe mit 1 bis 4 Kohlenstoffatomen oder $R_9$ und $R_{10}$ einen 5- oder 6-gliedrigen Ring bilden, der als weitere Heteroatome Stickstoff oder Sauerstoff enthalten kann, wobei der Ring durch wenigstens eine Alkylgruppe mit 1 bis 4 Kohlenstoffatomen, bevorzugt Methyl, substituiert sein kann,
X CH oder bevorzugt Stickstoff,
n im Fall von A = a eine der Zahlen 0 oder 1, besonders bevorzugt 0 und im Fall von A = b eine der Zahlen 0, 1, 2, 3 oder 4, bevorzugt 0, 1 oder 2,

bedeuten,
deren optisch aktive Verbindungen oder deren Racemate sowie gegebenenfalls deren physiologisch unbedenkliche Säureadditionssalze.

Soweit nichts anderes angegeben, bedeutet Halogen Fluor, Chlor, Brom oder Jod; ein Alkylrest eine verzweigte oder unverzweigte Alkylgruppe mit 1 bis 4 Kohlenstoffatomen, wie z.B. eine Methyl-, Ethyl-, n-Propyl-, iso-Propyl-, n-Butyl oder tert.-Butylgruppe, eine Arylgruppe bevorzugt die Phenylgruppe, eine Aralkylgruppe bevorzugt die Benzylgruppe.

Die Abkürzung AS bedeutet eine Aminosäure oder ein Peptid. Aus den zuvor angegebenen allgemeinen Definitionen der Aminosäuren sind die folgenden Aminosäuren bevorzugt:
Aad, γ-Abu, ε-Aca, Ach, Acp, β-Aib, Δ-Ala, Ama, Apm, Apr, Arg, Asn, Asu, Cys, Gln, Glu, His, Hyl, Hyp, 3-Hyp, Ise, Lys, Nle, Nva, Pec, Phe, Phg, Pic, Pro, Tle, Pyr, Ser, Thr, Tyr oder Trp.

Erfindungsgemäß sind die nachfolgend genannten Aminosäuren ganz besonders bevorzugt:

Ala, β-Ala, Gly, Val, Met, Asp, Sar, Met ($O_2$) Aib, Abu, Ile, oder Leu.

Die hier angegebene Kurzschreibweise ist mit der in der Literatur verwendeten (z.B. Houben Weyl, Lehrbuch der organischen Chemie) identisch, bezieht sich jedoch nicht ausschließlich auf die L-Konfiguration, sondern auch auf die optisch aktiven D-Aminosäuren sowie auch auf deren Racemate.

Die Erfindung umfaßt ferner Verfahren zur Herstellung der neuen Verbindungen und pharmazeutischen Zusammensetzung, die diese Verbindungen enthalten.

Die neuen Verbindungen der allgemeinen Formel I können nach Analogieverfahren, wie sie aus der Peptidchemie (z.B. in Houben-Weyl, Methoden der organischen Chemie, Band 15, Georg Thieme-Verlag, Stuttgart, 1974) beschrieben sind, aus den entsprechenden Carbonsäuren der allgemeinen Formel IIIa oder IIIb

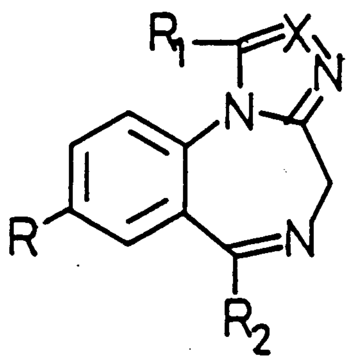

IIIa

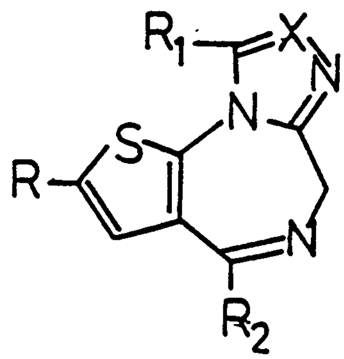

IIIb

worin die
Reste $R_1$, $R_2$ sowie X die angegebene Bedeutung haben und R eine Seitenkette der allgemeinen Formel

-$Y_n$-COOH

bedeutet,

a) durch Umsetzung mit einer Verbindung der allgemeinen Formel IV

H-<AS>$_m$-$R_8$

worin <AS>, $R_8$ und m die zuvor genannte Bedeutung aufweisen, in Gegenwart eines Carbodiimids, eines Carbonyldiimidazols, eines Sulfonyldiimidazols, oder

b) durch Überführung der freien Säure der allgemeinen Formel III in ein reaktives Säurederivat, wie z.B. ein Säurehalogenid, Säureimidazolid oder gemischtes Säureanhydrid und anschließende Umsetzung mit einer Verbindung der allgemeinen Formel IV.

Die Umsetzung der Säure mit der Verbindung IV erfolgt in Gegenwart eines Carbodiimids, beispielsweise Cyclohexylcarbodiimid, in einem inerten organischen Lösungsmittel, wie z.B. Dimethylformamid, Tetrahydrofuran, Dioxan und deren Mischungen, bei Temperaturen zwischen 0 ° C und dem Siedepunkt des Reaktionsgemisches, vorzugsweise zwischen O ° C und Raumtemperatur.

Die Reaktion des Aminobausteins der allgemeinen Formel IV mit einem Säurehalogenid, gemischten Anhydrid oder Säureimidazolid, hergestellt aus der allgemeinen Formel III, erfolgt in einem inerten organischen Lösungsmittel, wie z.B. Dimethylformamid, Tetrahydrofuran, Dioxan und Mischungen derselben, bei Temperaturen zwischen -20 ° C und dem Siedepunkt des Reaktionsgemisches, vorzugsweise zwischen O ° C und Raumtemperatur, wobei gegebenenfalls ein säurebindendes Mittel, wie z.B. Natriumcarbonat, Natriumbicarbonat oder eine tertiäre organische Base, beispielsweise Pyridin oder Triethylamin, zugegeben wird.

Die Kondensationsreaktionen können nach an sich bekannten Methoden durch Zugabe von N,N-Dimethylaminopyridin katalysiert werden.

Das Säurehalogenid bzw. gemischte Säureanhydrid der allgemeinen Formel III erhält man aus der freien Säure in üblicher Weise, z.B. durch Reaktion der Säure mit Oxalychlorid bzw. Umsetzung mit

Chlorameisensäureethylester.

Das Säureimidazolid erhält man in der üblichen Weise, z.B. durch Reaktion der Säure mit 1,1'-Carbonyldiimidazol.

Die neuen Verbindungen der allgemeinen Formel I, in denen $R_8$ eine Aminogruppe

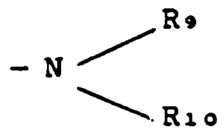

worin $R_9$ und $R_{10}$ die zuvor genannte Bedeutung aufweisen, bedeutet, können auch durch Umsetzung einer Verbindung der allgemeinen Formel V,

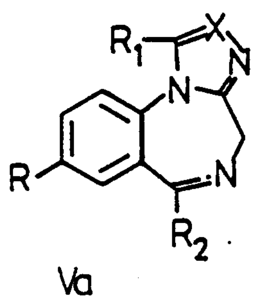

Va

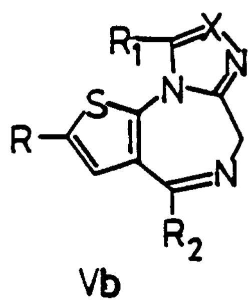

Vb

R = -$Y_n$-CO-<AS>$_m$-OH

mit einem Amin der allgemeinen Formel

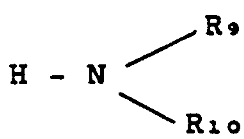

worin $R_9$ und $R_{10}$ wie oben definiert sind, nach bekannten Verfahren hergestellt werden.

Die freien Carbonsäuren der allgemeinen Formel V erhält man durch Verseifung von Verbindungen der allgemeinen Formel I, in denen $R_8$ Alkoxy- oder Alkylthio bedeutet, in üblicher Weise, z.B. durch alkalische Hydrolyse in einem Tetrahydrofuran/ Wassergemisch bei Raumtemperatur.

Die Aminosäureester, -amide, Peptidester und -amide der allgemeinen Formel IV erhält man nach in der Peptidchemie üblichen Verfahren, wie sie z.B. in Houben-Weyl, Band 15, beschrieben sind.

Die teilweise neuen Carbonsäuren der allgemeinen Formeln IIIa/b erhält man durch Verseifung der entsprechenden Ester VIa/b, z.B. mit Natriumhydroxid in einer Mischung aus Tetrahydrofuran und Wasser.

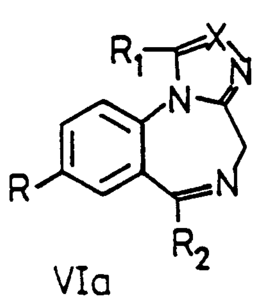

VIa

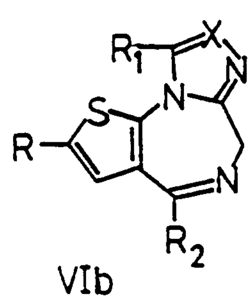

VIb

R = $-Y_n-COOR'$, R' = niederes Alkyl

Die Zwischenverbindungen der allgemeinen Formel VIa, b sind teilweise neu. Die Herstellung der Verbindungen VI, in denen X = N bedeutet, erfolgt nach bekannten Analogieverfahren.

Ausgehend von dem Thion VII

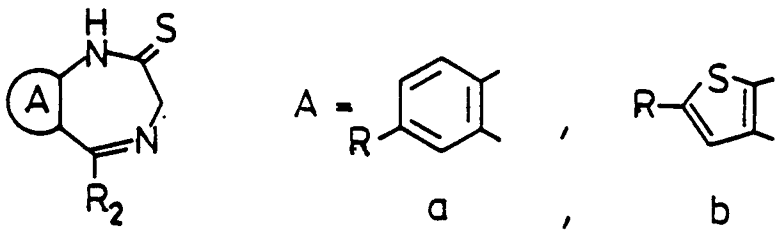

a , b

VII

R = $-Y_n-COOR'$.

erfolgt die Umsetzung entweder

a) mit einem Säurehydrazid der allgemeinen Formel VIII

$R_1CONHNH_2$

worin $R_1$ die zuvor genannte Bedeutung, mit Ausnahme von Halogen, aufweist, oder aber

b) mit Hydrazin in eine Verbindung der allgemeinen Formel IX

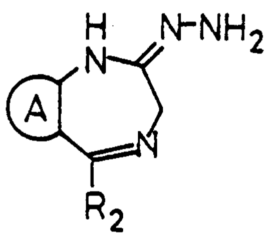

IX

überführt und anschließend mit einem Säurehalogenid der allgemeinen Formel

$$R_1-\overset{O}{\overset{\|}{C}}-Hal$$

worin Hal ein Halogenatom, bevorzugt Chlor, bedeutet und $R_1$ die zuvor genannte Bedeutung aufweist, oder mit einem Orthoester der allgemeinen Formel XI

$R_1C(OR'')_3$

worin R'' eine niedere Alkylgruppe bedeutet, umsetzt.

Die Umsetzung des Thions VII mit einem Säurehydrazid VIII nach dem Verfahren a) oder mit einem Hydrazin nach Verfahren b) erfolgt in einem inerten organischen Lösungsmittel, wie z.B. Dioxan, Dimethylformamid, Tetrahydrofuran oder einem geeigneten Kohlenwasserstoff, wie z.B. Benzol oder Toluol bei Temperaturen zwischen Raumtemperatur und dem Siedepunkt des Reaktionsgemisches.

Die dabei entstehenden Hydrazino-1,4-diazepine können nach herkömmlichen Verfahren isoliert oder aber auch direkt weiterverarbeitet werden.

Die weitere Umsetzung mit einem Säurehalogenid X oder einem Orthoester XI erfolgt in einem inerten organischen Lösungsmittel, wie z.B. halogenierte Kohlenwasserstoffe oder aliphatischen Ethern.

Die Isolierung der Endprodukte erfolgt nach bekannten Methoden, wie z.B. durch Kristallisation.

Die Herstellung von Verbindungen der allgemeinen Formel I, in denen X eine CH-Gruppe bedeutet, erfolgt aus den entsprechenden Carbonsäure der allgemeinen Formel III, worin $R_1$, $R_2$ und A die angegebene Bedeutung haben und X = CH ist, in der zuvor beschriebenen Weise. Verbindungen der allgemeinen Formel III bzw. VI mit X = CH, werden aus dem Thion der allgemeinen Formel VII durch Umsetzung mit einem Aminoalkin der allgemeinen Formel XII, worin $R_{11}$ Wasserstoff oder eine Alkylgruppe, bevorzugt Wasserstoff, bedeutet, erhalten.

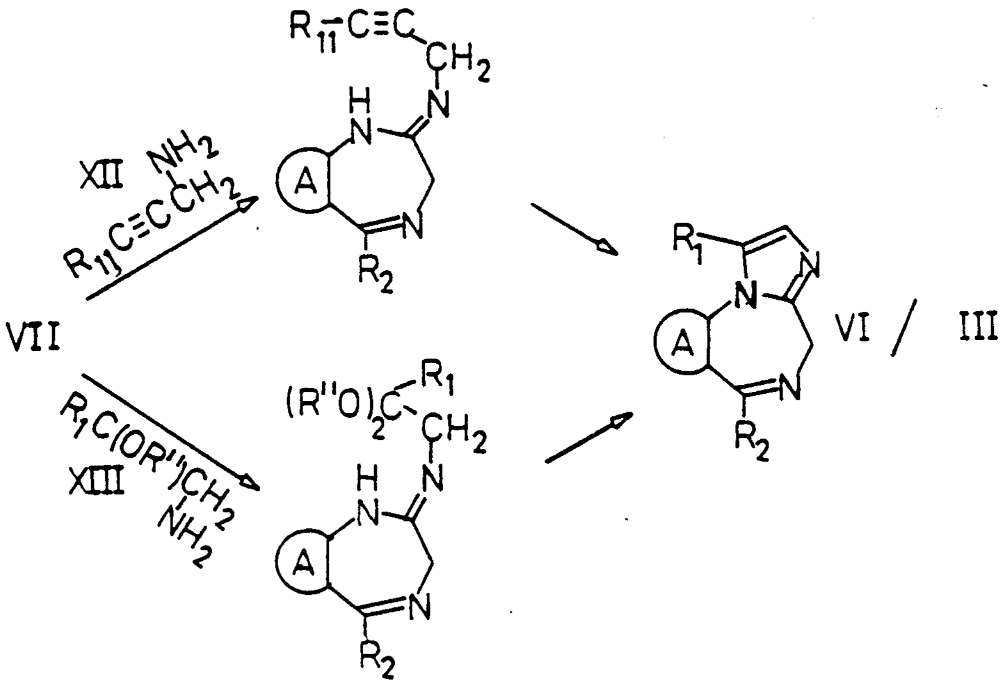


Nach diesem Verfahren können Verbindungen der allgemeinen Formel IIIa/b hergestellt werden, worin $R_1$ eine Alkyl, bevorzugt die Methylgruppe, bedeutet.

Ein weiteres Verfahren besteht in der Umsetzung des Thions der allgemeinen Formel VII mit einem alpha-Aminoaldehyd-alkylacetal oder -alkylketal der allgemeinen Formel XIII,

worin $R_1$ Wasserstoff oder eine Alkylgruppe mit 1 bis 4 Kohlenstoffatomen und R′ eine niedere Alkylgruppe bedeutet und $R_2$ und A die zuvor angegebene Bedeutung aufweisen.

Analogieverfahren zur Synthese eines Acetals oder Ketals der allgemeinen Formel XIII sowie ein Analogieverfahren zum Ringschluß sind in der Schweizerischen Patentschrift Nr. 580 099 und der DE-OS 23 21 705 beschrieben. Analogieverfahren zur Synthese des ankondensierten Imidazolringes sind ebenfalls in der DE-OS offenbart.

Unter den üblichen sauren Reaktionsbedingungen der Cyclisierung der Zwischenprodukte können, falls die funktionelle Seitenkette von A endständige Estergruppen aufweist, diese gleichzeitig in die gewünschten Carbonsäuren überführt werden.

Verbindungen der allgemeinen Formel I, in denen $R_1$ Chlor oder Brom bedeutet, werden beispielsweise entweder aus den Verbindungen der allgemeinen Formel I oder aber aus einer Verbindung der allgemeinen Formel III mit $R_1$ = Wasserstoff durch Umsetzung mit Chlor oder Brom in Pyridin hergestellt. Diese werden dann wie beschrieben in die Verbindungen der allgemeinen Formel I, mit $R_1$ = Cl, Br weiter überführt.

Die 1-Alkoxyverbindungen erhält man beispielsweise aus einer der zuvor erwähnten Chlor- bzw. Bromverbindung durch Umsetzung mit dem entsprechenden Alkoholat.

Eine weitere Methode zur Herstellung der Alkoxyverbindungen der allgemeinen Formel III ist beispielsweise die Umsetzung des Esters der allgemeinen Formel VI mit einem Halogen und anschließender Umsetzung mit dem entsprechenden Alkoholat.

Die Herstellung der Thione VII ist in Syntheseschema 3 beschrieben:

3)

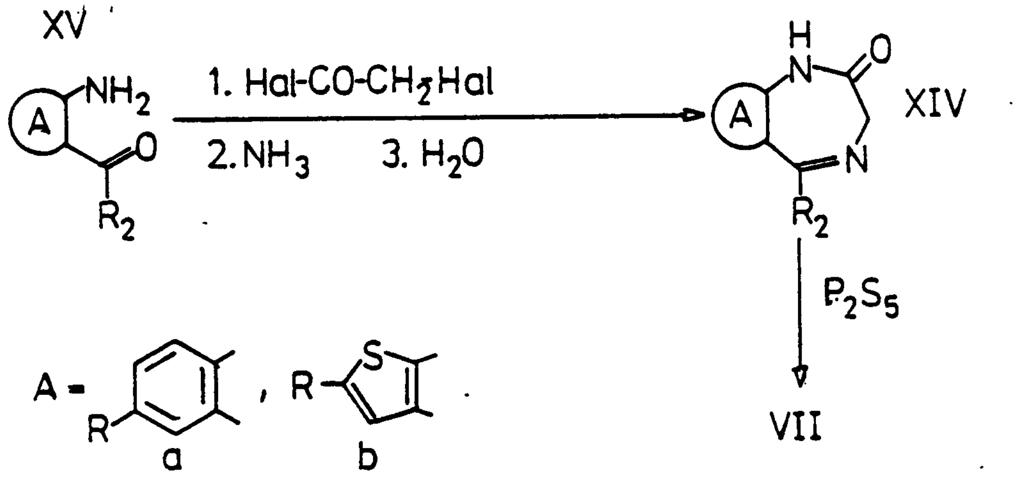


Die in dem Syntheseschema aufgeführten Zwischenverbindungen sind zum Teil bereits beschrieben oder aber im Analogieverfahren aus bekannten Verbindungen herstellbar. So ist beispielsweise die Synthese des Benzodiazepins XIVa, n = O, in Sternbach et al. Helv. Chim. Acta 186, 1720 (1969) beschrieben, die Synthese der Thienodiazepine XIVb für n = O aus der DOS 25 03 235, wie auch von W.D. Bechtel und K.H. Weber, J. Pharm. Sci, 74, 1265 (1985) für n = 2 bekannt.

Die Herstellung der Aminoketone der allgemeinen Formel XVb, worin $R_2$ die oben angegebene Bedeutung hat, ist in dem Syntheseschema 3′ beschrieben:

3')

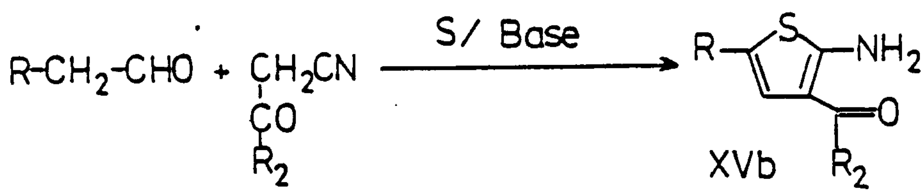


R kann die Bedeutung von R = $Y_n$-COOR′ oder-$Y_n$ -CH(COOR′)$_2$ haben.

Falls ein Dicarbonsäureester eingesetzt wurde, wird eine der Carboxylgruppen auf der Stufe des Aminoketons nach dem Verseifen abgespalten, wie es beispielsweise in W.D. Bechtel und K.H. Weber, J. Pharm.Sci. 74, 1265 (1985) beschrieben ist.

Die Synthese der Aminoketone XVa, worin $R_2$ wie zuvor definiert ist, ist in dem Syntheseschema 4, beispielsweise für n = O oder 1, dargestellt.

4)

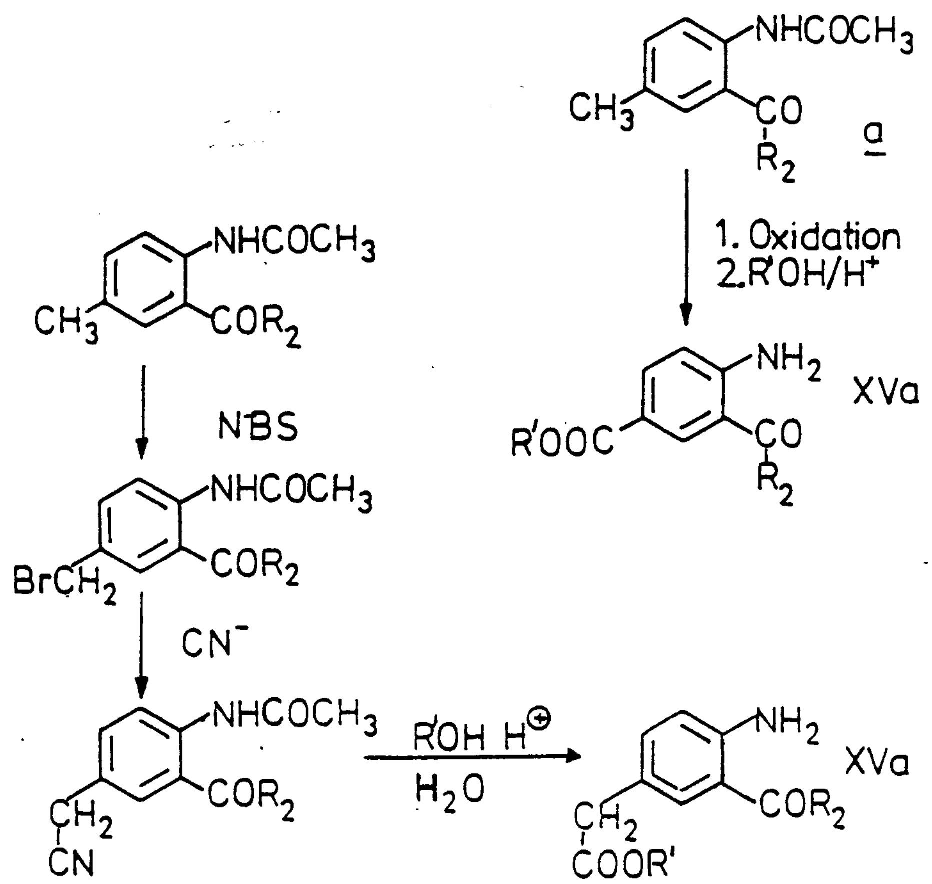


a

XVa

XVa

Die in Syntheseschema 4) dargestellten acetylierten Aminoketone (4) sind zum Teil bekannt; ihre Herstellung erfolgt nach Analogieverfahren und ist beispielsweise von D. Walsh in Synthesis, 1980, 677 - 688 beschrieben. Die Oxidation der Methylverbindung zur Säure erfolgt vorteilhafterweise mit Kaliumpermanganat in einem Pyridin/Wassergemisch bei Temperaturen zwischen 20 ° C und dem Siedepunkt des Reaktionsgemisches, bevorzugt bei 90 ° C.

Für n = 2 ist die Synthese beispielsweise bei M. Zinic et al., J. Heterocycl. Chem. 4, 1225 (1977) beschrieben.

Die Verbindungen der allgemeinen Formel I, in denen $R_8$ eine Alkoxygruppe bedeutet, können alternativ, wie in dem Syntheseschema 5 dargestellt, aus den Verbindungen der allgemeinen Formel XV, gegebenenfalls nach vorheriger Verseifung, hergestellt werden.

5)

XV

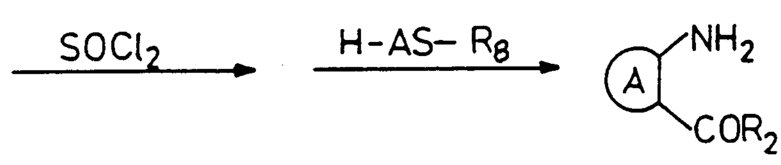


XVI

$R = -Y_n-COOH$ $R = -Y_n-CO-AS-R_8$

Die nach den oben genannten Verfahren hergestellte Säuren der Aminoketonstufe XV werden in das Säurehalogenid überführt, beispielsweise mit Thionylchlorid in einem inerten organischen Lösungsmittel zwischen Raumtemperatur und dem Siedepunkt des Reaktionsgemisches. Anschließend wird mit einen N-terminal deblockierten Aminosäureester der allgemeinen Formel IV, wie bereits oben beschrieben, umgestzt. Die erhaltenen Verbindungen der allgemeinen Formel XVI können dann wie zuvor beschrieben zu den Verbindungen der allgemeinen Formel I umgesetzt werden.

Die neuen Verbindungen der allgemeinen Formel II, in denen $R_3$ Wasserstoff ist, können in üblicher Weise aus den neuen Verbindungen I durch Reduktion mit bekannten Reduktionsmitteln gewonnen werden.

Die Reduktion erfolgt beispielsweise mit Zink in einer Mischung aus Eisessig und einem inerten organischen Lösungsmittel, wie beispielsweise halogenierte Kohlenwasserstoff, wie z.B. Dichlormethan, bei Temperaturen zwischen Raumtemperatur und dem Siedepunkt des Reaktionsgemisches. Verbindungen der allgemeinen Formel II, in denen $R_3$ eine Alkylgruppe bedeutet, lassen sich aus den zuvor genannten Verbindungen durch Alkylierung herstellen.

Nach einem der vorstehend beschriebenen Verfahren können beispielsweise die folgenden Verbindungen hergestellt werden:

N-{[1-Methyl-6-(2-chlorphenyl)-4H-[1,2,4]triazolo[4,3-a][1,4]benzodiazepin-8-yl-]carbonyl}-D, L-alanimethylamid.

N-{[1-Methyl-6-(2-chlorphenyl)-4H-[1,2,4]triazolo[4,3-a][1,4]benzodiazepin-8-yl-]carbonyl}-1-aminocyclohexan-1-carbonsäurediethylamid

N-{[1-Methyl-6-(2-chlorphenyl)-4H-[1,2,4]triazolo[4,3-a][1,4]benzodiazepin-8-yl-]carbonyl}-L-isoleucindiethylamid

N-{[1-Methyl-6-(2-chlorphenyl)-4H-[1,2,4]triazolo[4,3-a][1,4]benzodiazepin-8-yl-]carbonyl}-L-asparaginsäuredi-N′-methylamid

N-{[1-Methyl-6-(2-chlorphenyl)-4H-[1,2,4]triazolo[4,3-a][1,4]benzodiazepin-8-yl-]carbonyl}-DL-$\beta$-aminoisobuttersäuremorpholid

N-{2-[1-Methoxy-6-(2-chlorphenyl)-4H-[1,2,4]triazolo[4,3-a][1,4]benzodiazepin-8-yl]propionyl}-L-serinmorpholid

N-{2-[1-Methoxy-6-(2-chlorphenyl)-4H-[1,2,4]triazolo[4,3-a][1,4]benzodiazepin-8-yl]propionyl}-$\alpha$-methylalanindiethylamid

N-{2-[4-(2-Chlorphenyl)-9-methyl-6H-thieno[3,2-f]imidazo[1,2-a][1,4]diazepin-2-yl]propionyl}-glycinmorpholid

N-{2-[1-Methyl-6-phenyl-4H-[1,2,4]triazolo[4,3-a][1,4]benzodiazepin-8-yl]propionyl}-L-alanindiethylamid

N-{3-[4-(2-Chlorphenyl)-9-methyl-6H-thieno[3,2-f]imidazo[1,2-a][1,4]diazepin-2-yl]-2-methylpropionyl}-D,L-$\alpha$-aminopropionsäuredimethylamid

Die erfindungsgemäßen Verbindungen besitzen PAF-antagonistische Wirkung.

Bekanntlich handelt es sich bei PAF (Plättchen Aktivierender Factor) um das Phospholipid Acetyl-glycerylether-phosphoryl-cholin (AGEPC), das als potenter Lipidmediator bekannt ist, der von tierischen und menschlichen proinflammatorischen Zellen freigesetzt wird. Unter solchen Zellen finden sich hauptsächlich basophile und neutrophile Granulozyten, Makrophagen (aus Blut und Gewebe) sowie Thrombozyten, die an Entzündungsreaktionen beteiligt sind.

PAF zeigt im pharmakologischen Experiment Bronchokonstriktion, Blutdrucksenkung, Auslösung einer Thrombozytenaggregation sowie eine proinflammatorische Wirkung.

Diese experimentell nachweisbaren Wirkungen des PAF weisen direkt oder indirekt auf mögliche Funktionen dieses Mediators in der Anaphylaxie, in der Pathophysiologie des Asthma bronchiale und allgemein in der Entzündung hin.

PAF-Antagonisten werden benötigt, um einerseits weitere pathophysiologische Funktionen dieses Mediators an Tier und Mensch aufzuklären und andererseits pathologische Zustände und Krankheiten, an denen PAF beteiligt ist, zu behandeln. Beispiele für die Indikationen eines PAF-Antagonisten sind Entzündungsprozesse des Tracheobronchialbaumes (akute und chronische Bronchitis, Asthma bronchiale) oder der Niere (Glomerulonephritis), anaphylaktische Zustände, Allergien und Entzündungen im Bereich der Schleimhäute und der Haut (z.B. Psoriasis) sowie durch Sepsis, Endotoxine oder Verbrennungen bedingte Schockzustände. Weitere wichtige Indikationen für einen PAF-Antagonisten sind Läsionen und Entzündungen im Bereich der Magen- und Darmschleimhaut, wie z.B. Gastritis, im allgemeinen Ulcus pepticum, jedoch insbesondere Ulcus ventriculi und Ulcus duodeni.

Weiterhin eignen sich die erfindungsgemäßen Verbindungen zur Behandlung bei den folgenden Indikationsstellungen:

Obstruktive Lungenerkrankungen, wie z.B. bronchiale Hyperreaktivität, entzündliche Lungenwegserkrankungen, wie z.B. chronische Bronchitis;

Herz- Kreislauferkrankungen, wie z.B. Polytrauma, Anaphylaxe, Arteriosklerose, entzündliche Darmerkrankungen, EPH-Gestose (edima-protein uria Hypertension), Erkrankungen des extrakorporalen Kreislauf, ischämische Erkrankungen, entzündliche und immunologische Erkrankungen, Immunmodulation bei Tranplantationen von Fremdgeweben, Immunodulation bei Leukämie.

Metastasenausbreitung z.B. bei bronchialer Neoplasie, Erkrankungen des ZNS, wie z.B. Migrane,

Agarosephobie (panic disorder), weiterhin erweisen sich die erfindungsgemäßen Verbindungen als Cyto- und Organoprotektiv, z.B. zur Neuroprotektion, z.B. bei Leberzirrhose, DIC (desiminierte intravasale Gerinnung);
PAF assoziierte Interaktion mit Gewebshormonen (autocoid hormones), Lymphokine und anderen Mediatoren.

Die PAF-antagonistische Wirkung einzelner Benzodiazepine ist bekannt, vgl. E. Kornecki et al, Science 226, 1454 - 1456 (1984). Für Alprazolam wurde nach der unten beschriebenen Methode ein $IK_{50}$ - (Konzentration bei einer 50%igen Aggregationshemmung) von 14 $\mu$M, für Triazolam ein $IK_{50}$ von 9 $\mu$M gemessen. Diese, als Tranquilizer beziehungsweise Hypnotika ausgewiesenen und im Handel befindlichen Verbindungen sind jedoch wegen ihrer ausgeprägten sedierenden Wirkung trotz ihrer guten PAF-antagonistischen Wirkung zum Einsatz als PAF-Antagonisten in der Therapie in vielen Fällen ungeeignet.

Bei den erfindungsgemäßen Verbindungen hingegen fehlt die sedierende Wirksamkeit, während die PAF-antagonistische Wirkung im Vergleich zu der der bekannten Benzodiazepine wesentlich besser ist.

Die PAF-antagonistische Wirksamkeit einiger Verbindungen der Formeln I und II wurde anhand der Hemmung der Blutplättchen-Aggregation in vitro geprüft.

1. In vitro Untersuchungen: Hemmung der Blutplättchen-Aggregation

Zur Bestimmung der PAF-antagonistischen Wirkung von Substanzen wurde die durch PAF induzierte Aggregation von Humanthrombozyten in vitro verwendet. Zur Gewinnung von thrombozytenreichem Plasma (TRP) erfolgt die Blutentnahme aus einer nicht gestauten Vene mit Hilfe einer Plastikspritze, in der sich 3,8 %ige Natriumcitratlösung befindet. Das Verhältnis zwischen Natriumcitratlösung und Blut beträgt 1:9. Nach vorsichtiger Durchmischung wird das Citratblut bei 150 $\times$ g (1200 U/min) 20 Minuten lang zentrifugiert. Die Messung der Thrombozytenaggregation erfolgt nach dem von Born und Cross ausgearbeiteten Verfahren (G. V. R. Born und M.J. Cross, J. Physiol. 168, 178 (1963)), wobei dem TRP unter ständigem Rühren PAF als Auslöser der Aggregation zugesetzt wird.

Die Prüfsubstanz wird jeweils 2 - 3 Minuten vor Auslösung der Aggregation in einem Volumen von 10 $\mu$l zugesetzt. Als Lösungsmittel dienen entweder Aqua.dest., Ethanol und/oder Dimethylsulfoxyd. Kontrollansätze erhalten entsprechende Volumina dieser Lösungsmittel. Nach Registrierung der Ausgangsabsorption (2-3 Minuten) wird die Aggregation mit PAF (5 $\times$ $10^{-8}$ M) induziert.

Zur Beurteilung von Substanzeffekten wird das Maximum der ersten Aggregationswelle verwendet. Die durch PAF induzierte maximale Absorptionsrate (= maximale Aggregation $\times$ 100 %) wird jeweils gleichzeitig in einem Parallelansatz (= Kontrollansatz in einem der Kanäle des 2 Kanal-Aggregometers) zu jedem Testansatz (zweiter Kanal) mitgeprüft und als 100 %-Wert verwendet.

Der unter dem Einfluß der Testsubstanz erreichte Aggregationswert wird als 100 % angegeben.

Jede Prüfsubstanz wird bei Konzentrationen von $10^{-3}$ bis $10^{-8}$ M mit einem Stichprobenumfang von jeweils n = 4 hinsichtlich einer hemmenden Wirkung auf die durch PAF induzierte Thrombozytenaggregation untersucht. Danach wird eine Konzentrations-Wirkungskurve anhand von 3 Konzentrationen erstellt and die $IK_{50}$ (Konzentration bei einer 50%igen Aggregationshemmung) ermittelt. Die IK-Werte von Verbindungen der allgemeinen Formeln I oder II bewegen sich im allgemeinen um Werte, die kleiner als 9 $\mu$m sind.

In der Tabelle A sind die in vitro Untersuchungen bezüglich der Hemmung der Blutplättchenaggregation aufgeführt, wie es zuvor beschriebenist.

Tabelle A:

| Verbindung | $IK_{50}$ µ Mol |
|---|---|
| Alprazolam | 14 |
| Triazolam | 9 |
| Beispiel Nr. der Tabelle I | |
| 2 | 0.9 |
| 4 | 0.7 |
| 5 | 1.2 |
| 11 | 3.5 |
| 12 | 1.1 |
| 15 | 0.9 |
| 16 | 0.4 |
| 18 | 8.1 |
| 21 | 1.9 |
| 27 | 0.7 |
| 28 | 1.9 |
| 33 | 2.5 |
| 35 | 0.9 |
| 48 | 0.6 |

Die neuen Verbindungen der allgemeinen Formeln I oder II können warmblütigen Tieren topisch, oral, parenteral oder durch Inhalation verabreicht werden. Die Verbindungen liegen hierbei als aktive Bestandteile in üblichen Darreichungsformen vor, z.B. in Zusammensetzungen, die im wesentlichen aus einem inerten pharmazeutischen Träger und einer effektiven Dosis des Wirkstoffes bestehen, wie z.B. Tabletten, Dragees, Kapseln, Oblaten, Pulver, Lösungen, Suspensionen, Inhalationsaerosole, Salben, Emulsionen, Sirupe, Suppositorien usw.. Eine wirksame Dosis der erfindungsgemäßen Verbindungen liegt bei oraler Anwendung zwischen 1 und 200, vorzugsweise zwischen 20 und 100 mg/Dosis, bei intravenöser oder intramuskulärer Anwendung zwischen 0,01 und 50, vorzugsweise zwischen 0,1 und 10 mg/Dosis. Für die Inhalation sollen Lösungen, die 0,01 bis 1,0, vorzugsweise 0,1 bis 0,5 % Wirkstoff enthalten, eingesetzt werden.

Die folgenden Beispiele dienen zur näheren Erläuterung der Erfindung:

Beispiel 1

N-{[1-Methyl-6-(2-chlorphenyl)-4H-[1,2,4]triazolo[4,3-a][1,4]benzodiazepin-8-yl]carbonyl}glycinmorpholid

1.1. 2,-6-Dimethyl-4H-3,1-benzoxazin-4-on

45,5 g (0,33 mol) 5-Methylanthranilsäure werden zusammen mit 130 g Essisäureanhydrid 3 Stunden unter Rückfluß erwärmt. Nach Einengen des Lösungsmittels im Vakuum löst man den Rückstand in 200 ml Dichlormethan und filtriert über Kieselgel. Das eingeengte Filtrat wird mit Isopropylether zur Kristallisation gebracht.

Ausbeute: 48 g (84 % d.Th.), Fp. 124-125 °C

1.2. 2-Acetamido-5-methyl-2'-chlorbenzophenon

Das Grignard-Reagenz, hergestellt aus 312,5 g (1,63 mol) 2-Bromchlorbenzol und 39,6 g (1,63 mol) Magnesium in 1,2 Liter wasserfreiem Ether, tropft man bei Raumtemperatur unter Rühren innerhalb von 2,5 Stunden zu einer Lösung von 270 g (1,54 mol) 2,6-Dimethyl-4H-3,1-benzoxazin-4-on in 4 Liter

absolutem Ether. Man läßt noch 2 Stunden bei gleicher Temperatur nachreagieren. Unter Eiskühlung wird vorsichtig mit 2N-Salzsäure hydrolisiert und anschließend die wäßrige Phase abgetrennt. Die organische Phase wird zuerst mit verdünnter Natronlauge, dann mit Wasser gewaschen. Die organische Phase wird getrocknet und eingeengt. Der erhaltene Rückstand wird mit Isopropylether zur Kristallisation gebracht.

Ausbeute: 194 g (44 % d.Th.), Fp. 156-158°C (Fp. 158°C,

Eine alternative Synthese des 2-Acetamido-5-methyl-2'-chlorbenzophenons erfolgt in Analogie zu Sternbach et al., Helv. Chim. Acta 186, 1720 (1963)

Analog einem Literaturverfahren [JACS 78, 4842 (1978)] leitet man 82,5 g Bortrichlorid unter Eiskühlung in 350 ml Dichlorethan ein. Dazu tropft man innerhalb von 30 min 68 g 4-Methylanilin, gelöst in 400 ml Dichlorethan. Zu der Reaktionsmischung ergibt man dann 175 g 2-Chlorbenzonitril (Innentemp. 0°C) und anschließend portionsweise 94 g Aluminiumchlorid, wobei die Temp. auf 20 C ansteigt, dann erhitzt man 6 Stunden unter Rückfluß. Nach dem Abkühlen zersetzt unter Kühlen im Eisbad mit 400 ml 2N Salzsäure, erhitzt die Mischung für 30 min auf 80 C und extrahiert nach Abkühlen mit Dichlormethan. Nach dem Trocknen und Einengen der organischen Phase chromatogrphiert man den Rückstand and Kieselgel mit Laufmittel Dichlormethan/Methanol (98:2) und erhält 67,5 g 2-Amino-5-methyl-2'-chlorbenzophenon als Rohprodukt. Dies wird zusammen mit 22 g Acetylchlorid und 23 g Triethylamin in 600 ml Dichlormethan acetyliert. Nach der Aufarbeitung erhält man die Titelverbindung in einer Ausbeute von 57,1 g vom Fp. 156-158°C.

1.3. 4-Acetamido-3-(2-chlorbenzoyl)-benzoesäure

Man suspendiert 159,2 g (0,533 mol) 2-Acetamido-5-methyl-2'-chlorbenzophenon in einer Mischung aus 550 ml Pyridin und 1100 ml Wasser erwärmt auf 90°C und gibt unter kräftigem Rühren innerhalb von 3 Stunden 226,9 g (1,44 mol) Kaliumpermanganat portionsweise zu. Anschließend wird die Reaktionsmischung heiß filtriert und nach dem Abkühlen mehrfach mit Essigester extrahiert. Die wäßrige Phase wird mit Salzsäure auf pH=3 eingestellt und die dabei ausfallenden Kristalle werden isoliert.

Ausbeute: 103,5 g (59 % d.Th.), Fp. 272-273°C. (Sternbach et al., Fp. 263-265°C)

1.4. 4-Amino-3-(2-chlorbenzoyl)-benzoesäuremethylester

Es werden 103,5 g (0,326 mol) der zuvor hergestellten Benzoesäure in 1000 ml Methanol und 18,6 g para-Toluolsulfonsäurehydrat 24 Stunden unter Rückfluß erhitzt. Nach Einengen auf ein Viertel des Reaktionsvolumens werden die ausgefallenen Kristalle abgesaugt und zuerst mit Methanol, dann mit Isopropylether gewaschen.

Ausbeute: 84,5 g (90 % d.Th.), Fp. 155-156°C

1.5. 4-Bromacetamido-3-(2-chlorbenzoyl)-benzoesäuremethylester

84,5 g (0,29 mol) der oben beschriebenen Aminoverbindung werden in 800 ml wasserfreiem Dioxan gelöst und mit 24 ml Pyridin versetzt. Anschließend werden 26,3 ml (0.3 mol) Bromacetylbromid, gelöst in 100 ml wasserfreiem Dioxan, zugetropft. Nach 24 Stunden wird über Kieselgur filtriert, das Filtrat eingeengt und der Rückstand aus Aceton umkristallisiert.

Ausbeute: 100 g (84 % d.Th.), Fp. 136°C.

1.6. 1,3-Dihydro-5-(2-chlorphenyl)-2H-[1,4]benzodiazepin-2-on-7-carbonsäuremethylester

Zu 114 g (0,278 mol) der Bromacetylverbindung aus Beispiel 1.5 gelöst in 1500 ml Essigester, leitet man 3 Stunden bei Raumtemperatur Ammoniakgas ein. Man läßt anschließend 24 Stunden unter Rühren nachreagieren. Nach Filtration über Kieselgel und Einengen des Lösungsmittels erhält man die Aminoverbindung als Öl.

Das so erhaltene Rohprodukt wird in 1,2 Ltr. Toluol gelöst und nach Zugabe von 200 g $SiO_2$ für 1,5 Stunden am Wasserabscheider zum Rückfluß erhitzt. Aschließend wird filtriert und das entstandene Diazepinon mit einem erwärmten Methanol/Dichlormethan-Gemisch extrahiert. Die vereinigten Extrakte werden im Vakuum zur Trockne eingedampft und der Rückstand aus Essigester umkristallisiert.

Ausbeute: 77,9 g (91 % d.Th.), Fp. 245-248°C.

1.7. 1,3-Dihydro-5-(2-chlorphenyl)-2H-[1,4]benzodiazepin-2-thion-7-carbonsäuremethylester

37,5 g (0,114 mol) des oben genannten Benzodiazepinons werden in 350 ml Pyridin mit 32 g Phosphorpentasulfid 5 Stunden auf 65 °C erhitzt. Die Reaktionsmischung wird sodann in 700 ml einer 20%igen Kochsalzlösung eingerührt, dann mit Wasser verdünnt und das ausgefallene Benzodiazepin-2-thion abfiltriert und mit Ethanol gewaschen. Das Rohprodukt, 39 g, kann ohne weitere Reinigung weiter umgesetzt werden.

Umkristallisieren aus Isopropylether führt zum Thion vom Fp. 246-248°C.

1.8. 8-Carboxy-6-(2-chlorphenyl)-1-methyl-4H-[1,2,4]triazolo[4,3a][1,4]benzodiazepin

39 g (0,113 mol) des Thions werden in 500 ml Tetrahydrofuran mit 10 g Hydrazinhydrat versetzt und 30 Minuten bei Raumtemperatur gerührt. Die Suspension wird über Kieselgur filtriert und zur Trockne

eingeengt. Das Rohprodukt wird in 540 ml wasserfreiem Ethanol gelöst; nach Zugabe von 106 ml Orthoessigsäuretriethylester erhitzt man 1 Stunde auf Rückflußtemperatur. Nach Eindampfen des Lösungsmittels und Umkristallisieren aus Essigester erhält man 33 g des 6-(2-Chlorphenyl)-1-methyl-7-(methoxycarbonyl)-4H-[1,2,4]triazolo[4,3 a][1,4]benzodiazepins vom Fp. 169-173 ° C.
Eine analysenreine Substanz erhält man durch Umkristallisieren aus Methanol/Ether: Fp. 178-180 ° C.

Die Verbindung kann auch aus dem Benzodiazepin-2-thion mit Essigsäurehydrizid hergestellt werden.

33 g (0,09 mol) des obigen Esters werden mit 4,5 g (0,113 mol) Natriumhydroxid in einer Mischung aus 120 ml Wasser, 400 ml Tetrahydrofuran und 400 ml Methanol bei Siedetemperatur in 1 Stunde verseift. Man engt die Mischung ein, nimmt mit Wasser auf und säuert mit Eisessig an. Die ausgefallenen Kristalle werden isoliert und getrocknet.
Ausbeute: 28,6 g (90 % d.Th.), Fp. 350-352 ° C.

1.9. N-{[1-Methyl-6-(2-chlorphenyl)-4H-[1,24]triazolo[4,3-a][1,4]benzodiazepin-8-yl]-carbonyl}glycinmorpholid

1,75 g (5 mmol) 8-Carboxy-6-(2-chlorphenyl)-1-methyl-4H-[1,2,4]triazolo[4,3a][1,4]benzodiazepin werden in einem Lösungsmittelgemisch aus 40 ml wasserfreiem Tetrahydrofuran und 10 ml wasserfreiem Dimethylformamid vorgelegt. Anschließend gibt man 0,82 g (5 mmol) 1,1′-Carbonyldiimidazol zu und rührt 1 Stunde bei Raumtemperatur. Dann werden 0,56 g (5,5 mmol) Triethylamin und 1 g (5,6 mmol) Glycinmorpholid- hydrochlorid zugegeben. Nach 3 Tagen wird die Mischung eingeengt, der Rückstand in Dichlormethan/Wasser aufgenommen und mehrmals mit Wasser extrahiert. Die organische Phase wird eingeengt und der so erhaltene Rückstand mit Essigester verrieben.
Ausbeute: 2,1 g (87 % d.Th.) amorphe Substanz, Fp. 159-162 ° C.

Beispiel 2

N-{[1-Methyl-6-(2-chlorphenyl)-4H-[1,2,4]triazolo[4,3-a][1,4]benzodiazepin-8-yl]-acetyl}glycinmorpholid

2.1. 2-Acetamido-5-brommethyl-2′-chlorbenzophenon

52,8 g (0,18 mol) 2-Acetamido-5-methyl-2′-chlorbenzophenon werden in 185 ml wasserfreiem Tetrachlorkohlenstoff gelöst und in der Kälte mit 32 g (0,18 mol) N-Bromsuccinimid versetzt. Anschließend gibt man 0,4 g Azo-bis-isobutyronitril zu und erwärmt vorsichtig. Nach 8 Stunden Rühren bei Rückflußtemperatur wird filtriert, das Filtrat zweimal mit verdünnter Natronlauge und anschließend mit Wasser gewaschen. Die organische Phase wird eingeengt und der Rückstand aus Ethanol umkristallisiert.
Ausbeute 30 g (45 % d.Th.) der Bromverbindung, Fp. 108 ° C.

2.2. 4-Acetamido-3-(2-chlorbenzoyl)-phenylessigsäurenitril

65 g (0,18 mol) 2-Acetamido-5-brommethyl-2′-chlorbenzophenon werden in 85 ml wasserfreiem Triethylenglycol bei Raumtemperatur mit 9,8 (0,2 mol) Natriumcyanid versetzt und 1 Stunde gerührt. Zur Nachreaktion erwärmt man 1,5 Stunden auf 100 ° C. Nach dem Abkühlen wird mit 1000 ml Wasser versetzt, mehrfach mit Dichlormethan extrahiert und die organische Phase mit Wasser gewaschen, getrocknet und eingeengt. Der ölige Rückstand wird säulenchromatographisch über Kieselgel mit Dichlormethan/Methanol gereinigt.
Ausbeute: 32,2 g (58 % d.Th.) Öl.

2.3. 4-Amino-3-(2-chlorbenzoyl)-phenylessigsäureethylester

32,2 g (0,1 mol) 4-Acetamido-3-(2-chlorbenzoyl)-phenylessigsäurenitril werden in 340 ml wasserfreiem Ethanol gelöst. Unter Eiskühlung leitet man 1 Stunde trocknes Salzsäuregas ein. Anschließend wird 30 Minuten unter Rückfluß erhitzt und dann 24 Stunden bei Raumtemperatur gerührt. Mam versetzt die Reaktionsmischung mit der fünffachen Menge Wasser und läßt 15 Minuten stehen. Durch Extrahieren mit Dichlormethan, Trocknen und Einengen der organischen Phase und anschließender chromatographischer Aufarbeitung an Kieselgel erhält man 20,9 g (64 % d.Th.) der Titelverbindung als Öl

2.4. 4-Bromacetamido-3-(2-chlorbenzoyl)-phenylessigsäureethylester

20,9 g (0,066 mol 4-Amino-3-(2-chlorbenzoyl)-phenylessigsäureethylester werden in 170 ml wasserfreiem Dioxan gelöst. Man gibt zunächst 5,2 ml absolutes Pyridin hinzu und tropft dann 13,3 g (0,066 mol) Bromacetylbromid bei Raumtemperatur zu und rührt 24 Stunden. Die Suspension wird über Kieselgur filtriert. Man wäscht mit Ether nach und engt im Vakuum die vereinigten Filtrate ein. Der so erhaltene Rückstand wird durch Zugabe von Ethanol zur Kristallisation gebracht.
Ausbeute 24,6 g (85 % d.Th.), Fp. 83-85 ° C.

2.5. 5-(2-Chlorphenyl)-7-(ethoxycarbonylmethyl)-1,3-dihydro-2-oxo-2H-[1,4]-benzodiazepin

24,6 g (0,056 mol) der obigen Bromacetylverbindung werden in 280 ml Essigester gelöst. Man leitet 3

Stunden Ammoniakgas durch die Lösung und rührt zur Vervollständigung der Reaktion noch weitere 24 Stunden bei 25 ° C. Nach Filtration über Kieselgur werden die vereinigten Filtrate eingeengt, die dabei als Öl erhaltene Aminoverbindung wird direkt weiter umgesetzt.

Man versetzt das Öl mit 210 ml Toluol und 70 g Kieselgel und erhitzt die Reaktionsmischung 3 Stunden unter Rückfluß am Wasserabscheider. Anschließend wird filtriert, der Rückstand mit warmem Methanol mehrfach gewaschen und die gesammelten Filtrate zur Trockne eingeengt. Man reinigt durch chromatographische Aufarbeitung an Kieselgel mit Dichlormethan/Methanol (97 : 3) als Eluens und erhält das 1,4-Benzodiazepin-2-on als Öl.

Ausbeute: 19,3 g (97 % d.Th.)

2.6. 5-(2-Chlorphenyl)-7-(ethoxycarbonylmethyl)-1,3-dihydro-2H-[1,4]benzodiazepin-2-thion

Analog Beispiel 1 erhält man aus dem zuvor beschriebenen Benzodiazepinon das entsprechende Thion.

Ausbeute 13,3 g (66 % d.Th.)

Fp. 148-149 ° C.

2.7 6-(2-Chlorphenyl)-8-(ethoxycarbonylmethyl)-1-methyl-4H-[1,2,4]triazolo[4,3a][1,4]benzodiazepin

13,3 g (36 mmol) des obigen Thions werden wie in Beispiel 1 beschrieben zunächst in die Hydrazinoverbindung überführt und dann ohne weitere Reinigung mit Orthoessigsäuretriethylester umgesetzt. Man erhält nach chromatographischer Reinigung an $SiO_2$ mit Dichlormethan/Methanol (95/5) als Eluens die Triazoloverbindung.

Ausbeute 4,8 g; Fp. 156-157 ° C.

2.8 6-(2-chlorphenyl)-8-(carboxymethyl)-1-methyl-4H-[1,2,4]triazolo[4,3a][1,4]benzodiazepin

3,2 g (8 mmol) der Triazoloverbindung (Beispiel 2.7) wird wie in Beispiel 1 beschrieben verseift. Die bei pH = 3,5 isolierte Säure wird mit Isopropylether/Ether zur Kristallisation gebracht.

Ausbeute 2,3 g (77 % d.Th.) 6-(2-chlorphenyl)-8-(carboxymethyl)-1-methyl-4H- [1,2,4]triazolo[4,3a][1,4]-benzodiazepin

2.9 N-{[1-Methyl-6-(2-chlorphenyl)-4H-[1,2,4]triazolo[4,3-a][1,4]benzodiazepin-8-yl]-acetyl}glycinmorpholid

1 g (2,7 mmol) der nach 2.8 hergestellten 8-Carboxymethylverbindung setzt man mit 0.54 g (3mmol) Glycinmorpholidhydrochlorid wie in Beispiel 1 beschrieben und 1,1′-Carbonyldiimidazol um. Nach der Aufarbeitung erhält man durch Kristallisation aus Essigester/Ether 0,9 g (69 % d.Th.) der Titelverbindung vom Fp. 157-158 ° C als Semihydrat.

Beispiel 3

N-{[1-Cyclopropyl-6-(2-chlorphenyl)-4H-[1,2,4]triazolo[4,3-a][1,4]benzodiazepin-8-yl]carbonyl}-L-alaninpyrrolidid

3.1. 6-(2-Chlorphenyl)-l-cyclopropyl-8-(methoxycarbonyl)-[1,2,4]triazolo[4,3a][1,4]benzodiazepin

7 g (20,3 mmol) 5-(2-Chlorphenyl)-1,2-dihydro-7-(methoxycarbonyl)-2H-[1,4]benzodiazepin-2-thion und 3 g (30 mmol) Cyclopropancarbonsäurehydrazid erhitzt man 30 Stunden in 70 ml Dioxan unter Rückfluß. Anschließend wird das Lösungsmittel im Vakuum entfernt und der Rückstand chromatographisch an Kieselgel mit Toluol/Ethanol (9:1)

aufgearbeitet.Ausbeute: 2,9 g (36 % d.Th.) 6-(2-Chlorphenyl)-1- cyclopropyl-8-(methoxycarbonyl)-[1,2,4]-triazolo-[4,3a][1,4]benzodiazepin vom Fp. 185 ° C.

3.2 Die nachfolgende alkalische Hydrolyse wird wie in Beispiel 1 beschrieben durchgeführt. Nach Ansäuern mit Eisessig werden 2 g (90 % d.Th.) des 8-Carboxy-6-(2-chlorphenyl)-1-cyclopropyl[1,2,4]-triazolo[4,3a][1,4]benzodiazepins in kristalliner Form erhalten.

2 g (5,3 mmol) der so hergestellten Säure werden zusammen mit 0,8 g 1-Hydroxybenzotriazol, 0,6 g (6 mmol) Triethylamin und 1 g (5,6 mmol) L-Alaninpyrrolidid-Hydrochlorid in 30 ml N,N-Dimethylformamid vorgelegt. Nach Abkühlen auf 0 ° C fügt man 1,4 g (6,8 mmol) Dichyclohexylcarbodiimid zu und läßt 24 Stunden rühren. Es wird vom ausgefallenen Dicyclohexylharnstoff abfiltriert, das Filtrat zur Trockne eingeengt und der Rückstand mit Dichlormethan/Wasser aufgenommen. Die organische Phase extrahiert man nacheinander mit Natriumhydrogencarbonatlösung und mehrfach mit Wasser. Sodann wird die organische Phase mit Magnesiumsulfat getrocknet und der Rückstand nach Einengen aus Ether/Essigester umkristallisiert.

Ausbeute 2,6 g (96 % d.Th.) der Titelverbindung als Semihydrat vom Fp. 170-175 ° C, $[\alpha]_D^{20}$ = +20,5 ° (c = 1, $CHCl_3$)

Beispiel 4

N{[1-Methyl-6-(2-chlorphenyl)-4H-imidazo[1,2-a][1,4]benzodiazepin-8-yl]carbonyl}-β-alaninmethylester

4.1. 8-Carboxy-6-(2-chlorphenyl)-1-methyl-4H-imidazo[1,2-a][1,4]benzodiazepin
Zu 3,1 g (9 mmol) 5-(2-Chlorphenyl)-7-methoxycarbonyl)-[1,4]benzodiazepin-2-thion in 60 ml wasserfreiem Dioxan tropft man 1,6 g Propargylamin und erhitzt anschließend 3 Stunden unter Rückfluß und rührt eine weitere Stunde bei Raumtemperatur. Der nach Abziehen des Lösungsmittels verbliebene Rückstand wird in Dichlormethan/Wasser aufgenommen. Die wäßrige Phase wird nochmals mit Dichlormethan extrahiert, die gesammelten organischen Phasen mit Wasser gewaschen, das Lösungsmittel entfernt und der Rückstand aus Essigester/Ether umkristallisiert. Man erhält 2,1 g
5-(2-Chlorphenyl)-7-(methoxycarbonyl)-4H-2-propargylamino-[1,4]benzodiazepin vom Fp. 202-205° C.

1,6 g (4,37 mmol) der so erhaltenen Verbindung werden in 7,5 ml konzentrierter Schwefelsäure 10 Minuten auf 100° C erhitzt. Nach dem Abkühlen gießt man auf Eis, alkalisiert mit Ammoniaklösung und extrahiert mit Dichlormethan.
Die wäßrige Phase wird auf pH = 5 eingestellt und das ausgefallene 8-Carboxy-6-(2-chlorphenyl)-1-methyl-4H-imidazo[1,2-a][1,4]benzodiazepin abfiltriert und getrocknet.
Ausbeute: 1 g (65 % d.Th.); Fp. 295-297° C.

Die organische Phase wird eingeengt. Man erhält 0,2 g (13 % d.Th.) 8-Methoxycarbonyl-6-(2-chlorphenyl)-1-methyl-4H-imidazo[1,2-a][1,4]benzodiazepin vom Fp. 150-151° C.
4.2. 1 g (2,9 mol) der Imidazobenzodiazepincarbonsäure werden analog Beispiel 1 mit 1,1'-Carbonyldiimidazol und 0,42 g (3 mmol) β-Alaninmethylesterhydrochlorid umgesetzt und aufgearbeitet.
Man erhält 0,25 g (20 % d.Th.) der Titelverbindung vom Fp. 226-227° C.

## Beispiel 5

N-{[4-(2-Chlorphenyl)-9-methyl-6H-thieno[3,2-f][1,2,4]triazolo[4,3-a][1,4]diazepin-2-yl]-carbonyl}glycinethylester

5.1. N-{[5-Amino-4-(2-chlorbenzoyl)-thiophen-2-yl]carbony}glycinethylester
14,0 g (50 mmol) 5-Amino-4-(2-chlorbenzoyl)-thiophen-2-carbonsäure [Die Synthese erfolgt analog Hromatka et al., Monatsh. Chem. 104, 973 (1973)], werden in 250 ml wasserfreiem Dichlormethan suspendiert und nach Zutropfen von 8 ml Thionylchlorid 2,5 Stunden bei Raumtemperatur gerührt. Man dampft im Vakkum ein, nimmt den Rückstand in Dichlormethan auf und tropft die Lösung zu einer Mischung aus 7 g (50 mmol) Glycinethylesterhydrochlorid und 16 ml Triethylamin in 100 ml wasserfreiem Dichlormethan. Nach 1 Stunde bei Raumtemperatur extrahiert man mit Wasser. Der Rückstand der eingeengten organischen Phase wird chromatographisch an Kieselgel aufgearbeitet. Man erhält 8,1 g (44 % d.Th.) der Titelverbindung vom Fp. 216-218° C.
5.2 N-{[4-(2-Chlorphenyl)-thieno[2,3-b][1,4]diazepin-7-on-2-yl]carbonyl}glycinethylester
8,1 g (22,1 mmol) der nach 5.1 hergestellten Aminoverbindung werden anlog Beispiel 1 in 71 ml Dioxan und 1,8 ml Pyridin durch Zugabe von 2,3 ml (26,2 mmol) Bromacetylbromid in die Bromacetylverbindung überführt.
Ausbeute 9,9 g.

Das Rohprodukt wird in 170 ml Essigester gelöst, sodann wird in die Lösung 2 Stunden lang Ammoniak eingeleitet und wie in Beispiel 1 beschrieben aufgearbeitet. Man erhält die Aminoacetamidoverbindung als Öl, Ausbeute 8 g.

Die weitere Umsetzung erfolgt in Analogie zu Beispiel 1.6. Man erhält das im Titel genannte Thienodiazepinon vom Fp. 240-242° C (Essigester/Ether) in einer Ausbeute von 3,1 g (29 % d.Th., bezogen auf die eingesetzte Aminoverbindung).
5.2. 3,1 g (7,6 mmol) des obigen Thionodiazepin-2-ons werden in 25 ml Pyridin suspendiert, mit 1,7 g (7,7 mmol) Phosphorpentasulfid versetzt und 1 Stunde bei 60° C gerührt. Nach Aufarbeitung wie in Beispiel 1.7 beschrieben, erhält man 2,3 g (72 % d.Th.) des Thienodiazepin-2-thions vom Fp. 220-222° C.
5.3. Man suspendiert das erhaltene Thion in 20 ml Tetrahydrofuran, gibt 0,3 ml Hydrazinhydrat zu und arbeitet nach 0,5 Stunden bei Raumtemperatur auf. Es wird abfiltriert, der Rückstand mit Tetrahydrofuran gewaschen und die gesammelten Filtrate im Vakuum eingeengt. Die durch Verreiben mit Ether amorph erhaltene Hydrazinoverbindung (2,2 g (97 % d.Th.)), wird in einer Mischung aus 12 ml Orthoessigester und 10 ml wasserfreiem Ethanol 1 Stunde bei 80° C gerührt. Das Lösungsmittel wird entfernt und der Rückstand an Kieselgel mit Dichlormethan/Essigester/Methanol (7:2:1) als Eluens chromatographiert. Die einheitlichen Fraktionen werden eingedampft und mit Essigester zur Kristallisation gebracht. Man erhält

1,2 g (52 % d.Th.) N-{[4-(2-Chlorphenyl)-9-methyl-6H-thieno[3,2-f][1,2,4]- triazolo[4,3-a][1,4]diazepin-2-yl]carbonyl}-glycinethylester vom Fp. 251-252 ° C.

Beispiel 6

N-{[1-Methyl-6-(2-chlorphenyl)-4H-[1,2,4]triazolo[4,3-a]benzodiazepin-8-yl]carbonyl}-β-alaninmorpholid

2,5 g (5,7 mmol) des N{[1-Methyl-6-(2-chlorphenyl)-4H-[1,2,4]triazolo[4,3-a]benzodiazepin-8-yl]-carbonyl}-β-alaninmethylesters, hergestellt analog Beispiel 1 werden in einer Mischung von 50 ml Tetrahydrofuran und 5 ml Wasser gelöst. Man verseift unter Rühren bei Raumtemperatur durch Zutropfen von 1N Natronlauge unter Kontrolle des Laugenverbrauchs mit Thymophthalein als Indikator. Man neutralisiert nach Beendigung der Reaktion mit Eisessig, engt das Lösungsmittel ein, nimmt den Rückstand in Wasser auf und säuert mit 0,1n Salzsäure an. Das freigesetzte N{[1-Methyl-6-(2-chlorphenyl)-4H-[1,2,4]triazolo[4,3-a] benzodiazepin-8-yl]carbonyl}-β-alanin wird abfiltriert.
Ausbeute: 2,1 g (84 % d.Th.) vom Fp. 165-167 ° C (Hydrat).

1,25 g (2,8 mmol) der so erhaltenen β-Alaninsäure werden in 30 ml Tetrahydrofuran und 9 ml Dimethylformamid vorgelegt und mit 0,90 g (5,6 mmol) 1,1′-Carbonyldiimidazol versetzt. Nach einer Stunde tropft man 0,35 g (4 mmol) Morpholin, gelöst in 20 ml Tetrahydrofuran zu, und läßt 3 Tage reagieren. Anschließende Aufarbeitung wie in Beispiel 1 beschrieben, ergibt die Titelverbindung, welche aus Essigester/Ether kristallin erhalten wird.
Ausbeute: 0,9 g (64 % d.Th.), Fp. 218 ° C.

Beispiel 7

N{[1-Methyl-6-(2-chlorphenyl)-4H-[1,2,4]triazolo[4,3-a][1,4]benzodiazepin-8-yl]carbonyl}glycinamid

1 g (2,35 mmol) N{[1-Methyl-6-(2-chlorphenyl)-4H-[1,2,4]triazolo[4,3-a][1,4]benzodiazepin-8-yl]-carbonyl}glycinmethylester, hergestellt analog Beispiel 1 werden in 50 ml methanolischer Ammoniaklösung 2 Tage bei 25 ° C gerührt. Anschließend wird das Lösungsmittel im Vakuum entfernt und der Rückstand aus Essigester/Ether umkristallisiert. Man erhält 0,7 g (73 % d.Th.) des Glycinamids vom Fp. 185-187 ° C.

Beispiel 8

N{-[1-Methyl-6-(2-chlorphenyl)-5,6-dihydro-4H-[1,2,4]triazol[4,3-a][1,4]benzodiazepin-8-yl]-carbonyl}glycinmorpholid-dihydrochlorid

1 g (2,1 mmol) N{[1-Methyl-6-(2-chlorphenyl)-4H-[1,2,4]triazolo[4,3-a][1,4]benzodiazepin-8-yl]-carbonyl}glycinmorpholid werden in einer Mischung aus 18 ml Dichlormethan und 18 ml Eisessig gelöst, man versetzt mit 0,7 g (11 mmol) Zinkstaub und läßt 16 Stunden bei Raumtemperatur reagieren. Die Suspension wird über Kieselgur filtriert und mit Dichlormethan nachgewaschen. Die vereinigten Filtrate werden mit verdünnter Ammoniaklösung in der Kälte alkalisch gestellt. Man trennt die organische Phase ab und extrahiert die wäßrige Phase noch zweimal mit Dichlormethan. Einengen der organischen Phase und Lösen des so erhaltenen Rückstandes in ethanolischer Salzsäure und Fällen mit Ether, ergibt 0,7 g (58 % d.Th.) der Titelverbindung vom Fp. 218-220 ° C (Hydrat).

Beispiel 9

N-Methyl-N{[1-methyl-6-(2-chlorphenyl)-4H-[1,2,4,]triazolo[4,3-a][1,4]benzodiazepin-8-yl]-carbonyl}glycinmorpholid

1,8 g (3,7 mmol) N-{[1-methyl-6-(2-chlorphenyl)-4H-[1,2,4] triazolo[4,3-a][1,4]benzodiazepin-8-yl]-carbonyl}glycinmorpholid werden in 100 ml einer Mischung (10:1) aus wasserfreiem Tetrahydrofuran und Dimethylformamid vorgelegt und mit 0,25 g einer Natriumhydriddispersion (60%ig in Öl) 1 Stunde bei Raumtemperatur gerührt. Nach Zutropfen von 0,57 g (4 mmol) Methyljodid, gelöst in 10 ml wasserfreiem Tetrahydrofuran, läßt man 24 Stunden bei Raumtemperatur reagieren. Man entfernt das Lösungsmittels im Vakuum und fügt vorsichtig Wasser zu. Anschließend wird mit Dichlormethan extrahiert, wobei nach der üblichen Aufarbeitung und nachfolgender chromatographischer Reinigung die Titelverbindung in Form des Semihydrats aus Essigester vom Fp. 267-269 ° C in einer Ausbeute von 0,5 g (27 % d.Th.) erhalten wird.

Beispiel 10

N-{[1-Methyl-6-(2-chlorphenyl)-5,6-dihydro-4H-imidazo[1,2-a][1,4]benzodiazepin-8-yl]-carbonyl}glycinmorpholid

Ausgehend von N-{[1-Methyl-6-(2-chlorphenyl)-4H-imidazo-[1,2-a][1,4]benzodiazepin-8-yl]-carbonyl}glycinmorpholid erfolgt die Reduktion wie in Beispiel 8 beschrieben. Man erhält das Hydrat der Titelverbindung vom Fp. 159-162 ° C.

Beispiel 11

N-Methyl-N-{4-(2-chlorphenyl)-9-methyl-6-H-thieno[3,2-f][1,2,4]triazolo[4,3-a][1,4]diazepin-2-yl]-carbonyl}glycinmorpholid

Ausgehend von N-{4-(2-chlorphenyl)-9-methyl-6-H-thieno[3,2-f][1,2,4]triazolo[4,3-a][1,4]diazepin-2-yl]-carbonyl}glycin-morpholiderfolgt die Alkylierung in Analogie zu Beispiel 9.
Man erhält aus Ether das amorphe Semihydrat der Titelverbindung vom Fp. 140-143 ° C in 43%iger Ausbeute.

Beispiel 12

N-{[1-Brom-6-(2-chlorphenyl)-4H[1,2,4]triazolo[4,3-a][1,4]benzodiazepin-8-yl]carbonyl}glycinmorpholid

0,8 g (1,7 mmol) N-{6-(2-chlorphenyl)-4H-[1,2,4]triazolo[4,3-a] [1,4]benzodiazepin-8-yl]-carbonyl}glycinmorpholid, hergestellt analog zu Beispiel 1 werden in 15 ml wasserfreiem Chloroform gelöst, man fügt 0,2 g Pyridin und anschließend 0,33 g Brom zu. Nach viertägigem Rühren bei Raumtemperatur verdünnt man mit Dichlormethan, extrahiert die organische Phase mehrfach mit Wasser, trocknet und engt ein. Säulenchromatographie an Kieselgel mit Dichlormethan/Methanol als Eluens ergibt die Titelverbindung vom Fp. 168-170 ° C in einer Ausbeute von 0,15 g.

Beispiel 13

N-{[1-Methyl-6-(2-chlorphenyl)-5-methyl-5,6-dihydro-4-H-[1,2,4]triazolo[4,3-a][1,4]benzodiazepin-8-yl]-carbonyl}glycinmorpholid-hydrochlorid

1g (2,1 mmol N{[1-Methyl-6-(2-chlorphenyl)-5,6-dihydro-4H-[1,2,4]triazolo[4,3-a][1,4]benzodiazepin-8-yl]carbonyl}glycinmorpholid werden zusammen mit 0,7 g Ameisensäure und 0,37 g Formalinlösung (37 %ig) 16 Stunden unter Rückfluß erhitzt. Die Reaktionsmischung wird angesäuert und mit Ether behandelt. Die wässrige Phase wird abgetrennt und mit konzentrierter Ammoniaklösung alkalisch gestellt. Die Base wird mit Dichlormethan extrahiert und nach Eindampfen der organischen Phase in das Hydrochlorid mit ethanolischer Salzsäure überführt. Man erhält 0,4 g (35 % d.Th.) des Dihydrodiazepins in Form des Hydrochlorid-Hydrats vom Fp. 180-185 ° C.

Beispiel 14

N-{[1-Methoxy-6-(2-chlorphenyl)-4H-[1,2,4]triazolo[4,3-a][1,4]benzodiazepin-8-yl]carbonyl}glycinmorpholid

14.1 1-Brom-5-(2 chlorphenyl)-7-(methoxycarbonyl)-4H-[1,2,4]triazolo[4,3-a][1,4]benzodiazepin
6,7 g (19 mmol) 5-(2-chlorphenyl)-8-methoxycarbonyl)-4H-[1,2,4] triazolo[4,3-a][1,4]benzodiazepin werden in 130 ml wasserfreiem Chloroform gelöst. Nacheinander tropft man 2,2 g (28 mmol) Pyridin und 3,7 g (23 mmol) Brom zu und rührt 4 Tage bei Raumtemperatur. Die Reaktionsmischung wird mehrfach mit Wasser extrahiert, die organische Phase getrocknet und das Lösungsmittel im Vakuum eingeengt. Die chromatographische Reinigung mit Dichlormethan/Methanol (97:3) als Eluens führt zu 2,2 g (27 % d.Th.) der Bromverbindung vom Fp. 171-172 ° C.

14.2. 1,9 g (4,4 mmol) der 1-Bromtriazolobenzodiazepinverbindung werden in 180 ml Methanol, in denen 2,3 g Kaliumhydroxid gelöst sind, 1 Stunde bei 60 ° C gerührt. Nach Abdampfen des Lösungsmittels im Vakuum löst man den Rückstand in Dichlormethan. Anschließend wird mit Wasser extrahiert. Die wäßrige Phase wird angesäuert und die erhaltene Isomerenmischung der 1-Methoxy- und 1-Hydroxy-8-carboxy-6-(2-chlorphenyl)-4H-[1,2,4]triazolo[4,3-a][1,4]-benzodiazepine wird abfiltriert und getrocknet. Ausbeute: 1,1 g.

Die Isomerenmischung wird in 15 ml Dimethylformamid mit 0,5 g (2,8 mmol) Glycinmorpholidhydrochlorid, 0,3 g (3 mmol) Triethylamin und 0,4 g 1-Hydroxybenzotriazol gerührt, auf O°C abgekühlt und mit 0,7 g (3,4 mmol Dicyclohexylcarbodiimid versetzt. Nach 24 Stunden wird analog Beispiel 3 aufgearbeitet. Durch Chromatographieren an Kieselgel unter Verwendung von Dichlormethan/Essigester/Methanol (70:25:5) als Eluens trennt man die Isomeren auf. Man erhält 0,25 g (19 % d.Th.) N-{[1-Hydroxy-6-(2 chlorphenyl)-4H-[1,2,4]triazolo[4,3-a] [1,4]benzodiazepin-8-yl]-carbonyl}glycinmorpholid vom Fp. 190-192°C, und 0,45 g der Titelverbinbung als Semihydrat vom Fp. 179-182°C.

Beispiel 15

N-{[1-Methyl-6-(2-chlorphenyl)-4H-[1,2,4]triazolo[4,3-a][1,4]benzodiazepin-8-yl]carbonyl}-L-methioninsulfonmorpholid

0,7 g (1,26 mmol) N-{[1-Methyl-6-(2-chlorphenyl)-4H-[1,2,4]triazolo[4,3-a] [1,4]benzodiazeipin-8-yl]-carbonyl}-L-methioninmorpholid werden in 30 ml wasserfreiem Dichlormethan gelöst und bei O°C fügt man 0,6 g m-Chlorperbenzoesäure zu. Nach 24 Stunden bei O°C gibt man erneut 0,5 g m-Chorperbenzoesäure zu und rührt noch 3 Stunden bei 25°C. Man verdünnt mit 100ml Dichlormethan, schüttelt nacheinander mit gesättigter Natriumbisulfitlösung, verdünnter Natriumcarbonatlösung und Wasser aus. Die organische Phase wird eingeengt und der Rückstand säulenchromatographisch gereinigt. Durch Kristallisation mit Ether erhält man 0,2 g (26 % d.Th.) des Sulfons als Hydrat vom Fp. 185-187°C.

Beispiel 16

N-{3-[4-(2-Chlorphenyl)-9-methyl-6H-thieno[3,2-f][1,2,4]-triazolo[4,3-a][1,4]diazepin-2-yl]-propionyl}glycinmorpholid

0,55 g (1,4 mmol) 2-(2-Carboxyethyl)-4-(2-chlorphenyl)-9-methyl-6-H-thieno-[3,2-f][1,2,4]triazolo-[4,3-a]-[1,4]diazepin suspendiert man in 10 ml wasserfreiem Tetrahydrofuran, fügt 0,23 g (1,4 mmol) 1,1'-Carbonyldiimidazol und 5 ml wasserfreies Dimethylformamid zu und rührt 1 Stunde. Zu der Reaktionsmischung gibt man nacheinander 0,26 g (1,5 mmol) Glycinmorpholidhydrochlorid, 0,15 g (1,5 mmol) Triethylamin und läßt 24 Stunden bei Raumtemperatur rühren. Nach Aufarbeitung wie in Beispiel 1 beschrieben, erhält man durch Umkristallisation aus Essigester 0,4 g (56 % d.Th.) der Titelverbindung vom Fp. 201-203°C.

Die Herstellung der Ausgangsverbindung, die entsprechende Carbonsäure, ist in W.D. Bechtel und K.H. Weber, J. Pharm. Sci. 74, 1265 (1985) beschrieben.

Beispiel 17

L-1-Methyl-6-(2-chlorphenyl)-8-[(hexahydroazepin-2-on-3-yl)-aminocarbonyl-4H-[1,2,4]triazolo[4,3-a][1,4]-benzodiazepin

3,5 g (0,01 mol) 8-Carboxy-6-(2-chlorphenyl)-1-methyl-[1,2,4]triazolo-[4,3-a][1,4]-benzodiazepin suspendiert man in 40 ml Dichlormethan. Unter Eiskühlung tropft man 2,5 g (0,02 mol) Oxalylchlorid zu und rührt 2 Stunden bei Raumtemperatur. Man engt ein und destilliert zweimal mit wasserfreiem Chloroform nach. Der kristalline Rückstand wird mit Petrolether verrieben und abgesaugt. Man erhält 3,7 g (100 %) 8-(Chlorcarbonyl)-6-(2-chlorphenyl)-1-methyl-[1,2,4]triazolo-[4,3-a][1,4]-benzodiazepin vom Fp. 295-297°C.

3,7 g (0,01 mol) Säurechlorid, 3,2 g (0,025 mol) L-2-Amino-$\epsilon$-caprolactam und 0,2 g (0,0016 mol) N,N-Dimethyl-4-aminopyridin rührt man 16 Stunden in 100 ml wasserfreiem Dichlormethan. Die Emulsion wird mit dem gleichen Lösungsmittel verdünnt, nacheinander mit verdünnter Ammoniaklösung und Wasser gewaschen. Nach Trocknen und Einengen nimmt man den Rückstand in Dichlormethan/Methanol auf und filtriert über wenig Kieselgel. Die eingedampften Filtrate werden mit Ether kristallisiert. Man erhält 2,9 g (63 % d.Th.) der Titelverbindung vom Fp. 317°C, $[\alpha]_D^{20}$ = + 38° (c=1, $CHCl_3$).

In Analogie zu den in den Beispielen beschriebenen Verfahren werden die folgenden Verbindungen der allegemeinen Formel I oder II hergestellt:

Tabelle I

| Nr. | Formel | X | $R_1$ | $R_2$ | $R_4$ | Fp°C,α |
|---|---|---|---|---|---|---|
| 1 | Ia | N | $-CH_3$ | 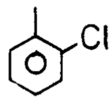 | O O<br>‖ ‖<br>$-C-NH-CH_2-C-OC_2H_5$ | 194 |
| 2 | Ia | CH | $-CH_3$ | " | O O<br>‖ ‖<br>$-C-NH-CH_2-CH_2-C-OCH_3$ | 226-227 |
| 3 | Ia | N | $-CH_3$ | " | O O<br>‖ ‖<br>$-C-NH-CH-C-OCH_3$<br>\|<br>$CH_2-CH_2-S-CH_3$ | 191-192<br>+ 11,1°<br>L |
| 4 | Ia | N | $-CH_3$ | " | 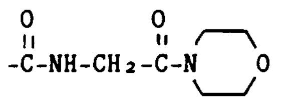 | 159-162<br>amorph |
| 5 | Ia | N | $-CH_3$ | " | O<br>‖<br>$-C-NH-CH-CO-OCH_3$<br>\|<br>$CH_2-CO-OCH_3$ | 175-176<br>+ 36,6°<br>L |
| 6 | Ia | N | $-CH_3$ | " | 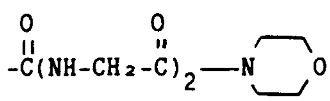 | amorph |
| 7 | Ia | N | $-CH_3$ | " | O O<br>‖ ‖<br>$-C-NH-CH_2-CH_2-C-OCH_3$ | 212 |
| 8 | Ia | N | $-CH_3$ | " | O O<br>‖ ‖<br>$-C-NH-CH_2-C-NH_2$ | 185-187 |
| 9 | Ia | N | $-CH_3$ | " | 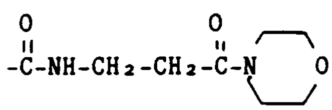 | 218 |

| Nr. | Formel | X | $R_1$ | $R_2$ | $R_4$ | Fp°C,α |
|---|---|---|---|---|---|---|
| 10 | Ia | N | $-CH_3$ | 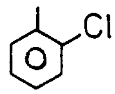 | 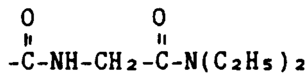 | 148-150<br>Semi-<br>hydrat |
| 11 | Ia | N | $-CH_3$ | " | 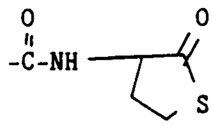 | 207-210<br>D,L |
| 12 | Ia | N | $-CH_3$ | " | 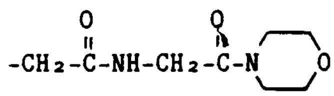 | 157-158<br>Semhdr. |
| 13 | Ia | N | $-CH_3$ | " | 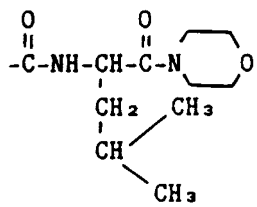 | 152-155<br>hydrat<br>+ 28,3°<br>L |
| 14 | Ia | N | $-CH_3$ | " | 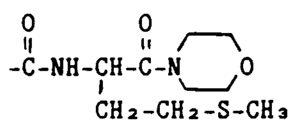 | 138-142<br>Semhydr<br>+ 24,3°<br>L |
| 15 | Ia | N | $-CH_3$ | " | 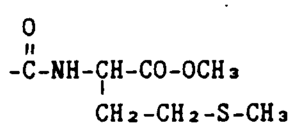 | 192-193<br>- 11,6°<br>D |
| 16 | Ia | N | $-CH_3$ | " | 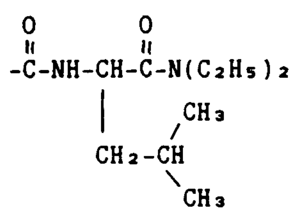 | 155-158<br>Semhydr<br>+ 11,8°<br>L |
| 17 | Ia | N | $-CH_3$ | " | 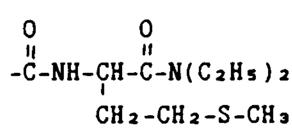 | 120-124<br>Hydrat |

| Nr. | Formel | X | $R_1$ | $R_2$ | $R_4$ | Fp°C,α |
|---|---|---|---|---|---|---|
| 18 | Ia | N | $-CH_3$ | 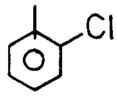 | 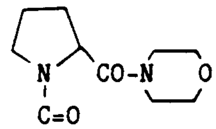 | 174-177 Hydrat - 24,5° L |
| 19 | Ia | N | $-CH_3$ | 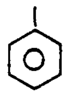 | 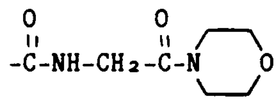 | 186-187 Hydrat |
| 20 | Ib | N | $-CH_3$ | 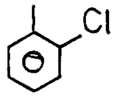 | 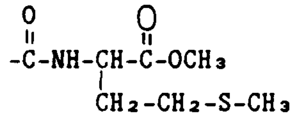 | 142-145 + 15,5° L |
| 21 | IIa | N | $-CH_3$ | " | 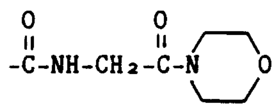 $R_3$=H | 218-220 x2HCl $xH_2O$ |
| 22 | Ia | N | $-CH_3$ | " | 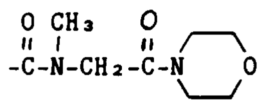 | 267-269 Semhydr |
| 23 | Ib | N | $-CH_3$ | " | 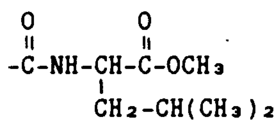 | 183-184 + 13,1° L |
| 24 | Ia | N | $-CH_3$ | " | 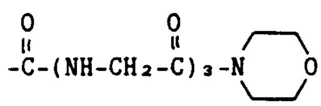 | amorph |
| 25 | Ia | N | $-CH_3$ | " | 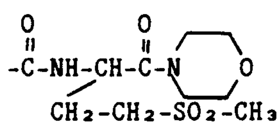 | 185-187 Hydrat + 23,2° L |

| Nr. | Formel | X | $R_1$ | $R_2$ | $R_4$ | Fp°C, α |
|---|---|---|---|---|---|---|
| 26 | Ia | N | $-CH_3$ | 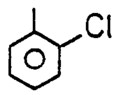 | 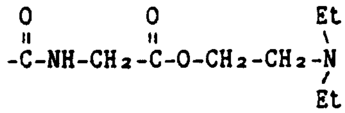 | 157-160 + 2HCl |
| 27 | Ia | N | $-CH_3$ | " | 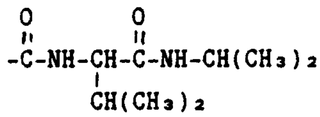 | 180-182 + 8,7° L |
| 28 | Ib | N | $-CH_3$ | " | 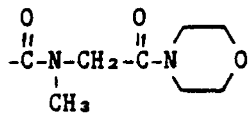 | 140-143 |
| 29 | Ia | N | $-CH_3$ | " | 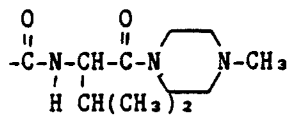 | 150-152 Semhydr + 43,5° L |
| 30 | Ia | N | $-CH_3$ | " | 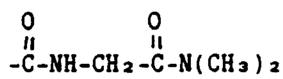 | 168-169 Semihydr |
| 31 | Ib | N | $-CH_3$ | " | 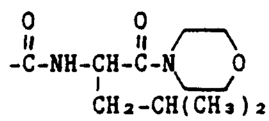 | 230-233 + 23° L |
| 32 | Ia | N | $-CH_3$ | " | 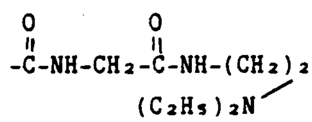 | 133-137 Hydrat |
| 33 | Ia | N | $-OCH_3$ | " | 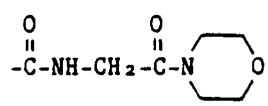 | 179-182 |
| 34 | IIa | N | $-CH_3$ | " | 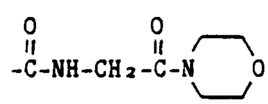 $R_3=CH_3$ | 180-185 Hydrat x 2HCl |

| Nr. | Formel | X | $R_1$ | $R_2$ | $R_4$ | Fp°C,α |
|---|---|---|---|---|---|---|
| 35 | Ia | N | 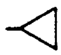 | 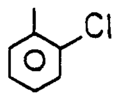 | 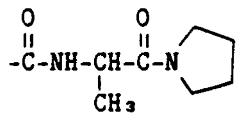 | 170-175 Semihydr + 20,5° L |
| 36 | Ia | CH | $-CH_3$ | " | 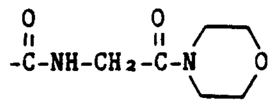 | 153-156 |
| 37 | IIa | CH | $-CH_3$ | " | 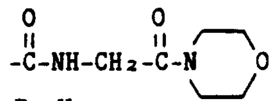 $R_3$=H | 159-162 Hydrat |
| 38 | Ia | N | $-CH_3$ | " | 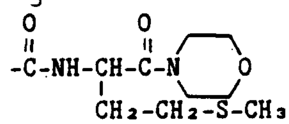 | 154-157 - 23,7° D |
| 39 | Ia | N | $-CH_3$ | " | 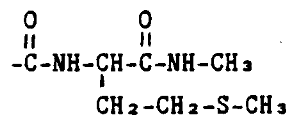 | amorph |
| 40 | Ia | N | -H | " | 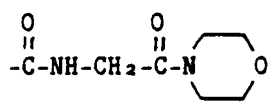 | 157-158 Hydrat |
| 41 | Ia | N | -Br | " | 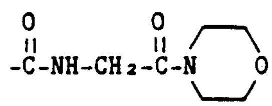 | 168-170 |
| 42 | Ia | N | $-CH_3$ | " | 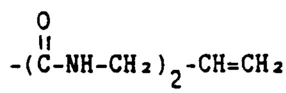 | 170-172 |
| 43 | Ib | N | $-CH_3$ | " | 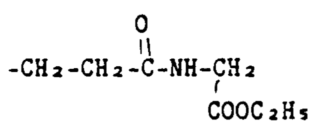 | 187-188 |
| 44 | Ib | N | $-CH_3$ | " | 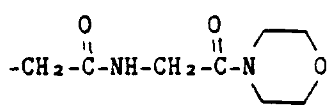 | amorph |

| Nr. | Formel | X | $R_1$ | $R_2$ | $R_4$ | Fp°C,α |
|---|---|---|---|---|---|---|
| 45 | Ib | N | $-CH_3$ | 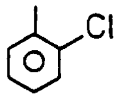 | 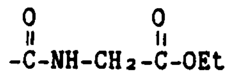 | 251-252 |
| 46 | Ib | N | $-CH_3$ | " | 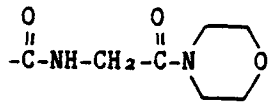 | 169-173 |
| 47 | Ib | N | $-CH_3$ | " | 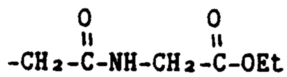 | 179-182 |
| 48 | Ib | N | $-CH_3$ | " | 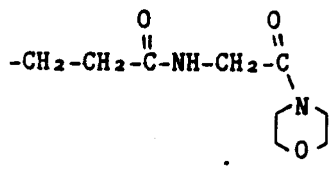 | 201-203 |
| 49 | Ia | N | $-CH_3$ | " | $-CO-NH-CH_2-COOCH_3$ | 158-159 |
| 50 | Ia | N | $-CH_3$ | " | $-CO-NCH_3-CH_2-COOCH_3$ | amorph |
| 51 | Ia | N | $-CH_3$ | " | 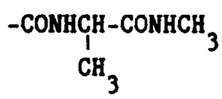 | 191-192 |

α bedeutet $[\alpha]^{20}$ und gibt den Drehwert der Verbindung an, L oder D die Konfiguration der Aminosäure an.

In der Tabelle steht Et für $C_2H_5$-.

Die folgenden Zwischenverbindungen werden beispielsweise nach den in den Beispielen beschriebenen Verfahren hergestellt:

| Formel | X | $R_1$ | $R_2$ | $R_4$ | Fp. |
|---|---|---|---|---|---|
| Ia | N | $CH_3$ | 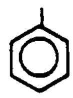 | $COOCH_3$ | 239-240°C |
| Ia | N | $CH_3$ | " | COOH | 352-355°C |
| Ia | N | H | 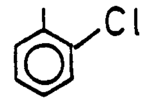 | $COOCH_3$ | 239-240°C |
| Ia | N | H | " | COOH | |
| Ia | N | $CH_3$ | " | $CONHCH_2COOH$ | 190-192°C Hydrat |
| Ib | N | $CH_3$ | " | $CONHCH_2COOH$ | 212-216°C |
| Ib | N | $CH_3$ | " | CO-L-Leu-OH | 185°C |

N-{[2-Amino-3-(2-chlorbenzoyl)-thiophen-5-yl]carbonyl}-L-methioninmethylester, Fp. 161-162° C.
N-{[2-Amino-3-(2-chlorbenzoyl)-thiophen-5-yl]carbonyl}L-leucinmethylester, Fp. 178° C.
N-{[2-Bromacetamido-3-(2-chlorbenzoyl)-thiophen-5-yl]carbonyl}-L-methioninmethylester, Fp. 122-125° C. (Zers.)
N-{[2-Bromacetamido-3-(2-chlorbenzoyl)-thiophen-5-yl]carbonyl}-L-leucinmethylester, Fp. 174-177° C.
N-{[4-(2-chlorphenyl)-thieno[4,2-f][1,4]diazepin-7-on-2-yl]carbonyl}-L-methioninmethylester, Fp. 191-193° C.
N-{[4-(2-chlorphenyl)-thieno[4,2-f][1,4]diazepin-7-on-2-yl]carbonyl-L-leucinmethylester, Fp. 227° C.

Weitere Thiophendiazepincarbonsäuren werden wie folgt erhalten:
2-Carboxy-4-(2-chlorphenyl)-9-methyl-6-H-thieno[3,2-f][1,2,4]triazolo[4,3-a][1,4]diazepin (Fp. 302° C) erhält man nach der in DOS 25 03 235 beschriebenen Methode;
2-Carboxymethyl-4-(2-chlorphenyl)-9-methyl-6-H-thieno[3,2-f][1,2,4]triazolo[4,3-a][1,4]diazepin ist auf folgendem Wege zugänglich:
Malonsäurediethylester und Bromacetaldehydacetal ergeben nach literaturbekannten Verfahren den Dicarbethoxypropionaldehyd (Kp 0,01 : 92-95° C), der sich nach dem für Beispiel 16 zitierten Literaturbeispiel mit Chlorcyanacetophenon und Schwefel in das entsprechende 2-Aminobenzoylthiophen umwandeln läßt. Verseifung, Decarboxylierung und Veresterung mit Methanol/Schwefelsäure ergeben das 2-Amino-3-(2-chlorbenzoyl)-5-(2-methoxycarbonyl-methyl)- thiophen. Bromacetylierung, Aminierung führten zum entsprechenden Diazepinon vom Fp. 180-182° C. Das hieraus hergestellte Thion schmilzt bei 184-185° C. Umsetzung mit Hydrazin und nachfolgende Ringschlußreaktion mit Orthoessigsäureester führt zum Triazolothienocarbonsäuremethylester vom Fp. 139-141° C. Dieser wird mit alkoholisch-wässriger Kalilauge zur freien Carbonsäure vom Fp. 257-259° C verseift.

4-[4-(2-Chlorphenyl)-9-methyl-6H-thieno[3,f][1,2,4]triazolo[4,3-a][1,4]diazepin-2-yl]-butan-1-carbonsäure

Ausgehend von Cycloheptanon ist nach literaturbekannter Methode (L. Claisen, Ber.dtsch.chem.Ges. 40, (3907) der Enolether zugänglich, den man einer Ozonolyse (V. Schmid, P. Grafen, Liebigs Ann. Chem. 656, 97 (1962) unterwirft.

Erhalten wird der 6-Formylheptansäuremethylester ($Kp_{15}$ = 115-170° C),der wie bereits beschrieben, zum 2-Amino-3-(2-chlorbenzoyl)-thiophen-2-butancarbonsäureester umgesetzt wird.

Den Thienotriazolo-1,4-diazepin-2-butancarbonsäuremethylester vom Fp. 119-121° C erhält man analog dem vorstehenden Beispiel. Durch Verseifung gewinnt man die Säure vom Fp. 113-134° C.

5-Phenyl-7-(1-carboxyethyl)-1,3-dihydro-2H-[1,4]benzodiazepine-2-on (Fp. 252-255° C) erhält man, wie bei M. Zinic et al., J. Heterocycl. Chem. 14, 1225 (1977) beschrieben.

Nachfolgend werden Beispiele für einige pharmazeutische Zusammensetzungen unter Verwendung von Verbindungen der allgemeinen Formel I oder II als aktiver Bestandteil angegeben. Falls nicht ausdrücklich

anders bezeichnet, handelt es sich bei den Teilen um Gewichsteile.

1. Tabletten

Die Tablette enthält folgende Bestandteile:

| | |
|---|---|
| Wirkstoff gemäß Formel I oder II | 0,020 Teile |
| Stearinsäure | 0,010 " |
| Dextrose | 1,890 " |
| gesamt | 1,920 Teile |

Herstellung:

Die Stoffe werden in bekannter Weise zusammengemischt und die Mischung zu Tabletten verpreßt, von denen jede 1,92 g wiegt und 20 mg Wirkstoff enthält.

2. Salbe

Die Salbe setzt sich aus folgenden Bestandteilen zusammen:

| | |
|---|---|
| Wirkstoff gemäß Formel I oder II | 50 mg |
| Neribas Salbe (Handelsware Scherax) | ad 10 g |

Herstellung:

Der Wirkstoff wird mit 0,5 g Salbengrundlage verrieben und die restliche Grundlage in Teilmengen zu 1,0 g nach und nach innig zu einer Salbe vermischt. Man erhält eine 0,5 %ige Salbe. Die Verteilung des Wirkstoffes in der Grundlage wird optisch unter dem Mikroskop kontrolliert.

3. Creme

Zusammensetzung:

| | | |
|---|---|---|
| Wirkstoff gemäß Formel I oder II | | 50 mg |
| Neribas Salbe (Handelsware Scherax) | ad | 10 g |

Herstellung

Der Wirkstoff wird mit 0,5 g Cremegrundlage verrieben und die restliche Grundlage in Teilmengen zu 1,0 g nach und nach mit Pistill eingearbeitet. Man erhält eine 0,5%ige Creme. Die Verteilung des Wirkstoffes in der Grundlage wird optisch unter dem Mikroskop kontrolliert.

4. Ampullenlösung

Zusammensetzung:.

| | | |
|---|---|---|
| Wirkstoff gemäß Formel I oder II | | 1,0 mg |
| Natriumchlorid | | 45,0 mg |
| Aqua pro inj. | ad | 5,0 ml |

Herstellung:
Der Wirkstoff wird bei Eigen-pH in Wasser gelöst und Natriumchlorid als Isotonanz zugegeben. Die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 1 mg, 5 mg und 1 mg Wirkstoff.

5. Suppositorien

Jedes Zäpfchen enthält:

| | |
|---|---|
| Wirkstoff gemäß Formel I oder II | 1,0 Teile |
| Kakaobutter (Fp.36-37°C) | 1200,0 Teile |
| Carnaubawachs | 5,0 Teile |

Herstellung
Kakaobutter und Carnaubawachs werden zusammengeschmolzen. Bei 45 °C gibt man den Wirkstoff hinzu und rührt, bis eine komplette Dispersion entstanden ist.
Die Mischung wird in Formen entsprechender Größe gegossen und die Zäpfchen zweckmäßig verpackt.

6. Inhalationslösungen

| | | | |
|---|---|---|---|
| a) | Wirkstoff gemäß Formel I oder II | | 500 mg |
| | Na-EDTA | | 50 mg |
| | Benzalkoniumchlorid | | 25 mg |
| | Natriumchlorid | | 880 mg |
| | destilliertes Wasser | ad | 100 ml |

Herstellung:
96 % der Wassermenge werden vorgelegt, darin nacheinander Na-EDTA, Benzalkoniumchlorid, Natriumchlorid und Wirkstoff klar gelöst und mit dem restlichen Wasser aufgefüllt. Die Lösung wird in 20 ml-Tropfflaschen abgefüllt. Eine Dosis (20 Tropfen, 1 ml) entält 5 mg Wirkstoff.

| | | | |
|---|---|---|---|
| b) | Wirkstoff gemäß Formel I oder II | | 500 mg |
| | Natriumchlorid | | 820 mg |
| | destilliertes Wasser | ad | 100 ml |

Herstellung
96 % der Wasserstoffmenge werden vorgelegt, darin nacheinander der Wirkstoff und Natriumchlorid gelöst, mit dem restlichen Wasser aufgefüllt und die Lösung in Eindosenbehälter (4 ml) abgefüllt. Die Lösung enthält 20 mg Wirkstoff.

Nachfolgend werden einige 1H-NMR Daten ausgewählter Verbindungen der Beispiele der Tabelle I aufgeführt:

Beispiel 4:

$^1$H-NMR ($CD_3OD$) $\delta$ (ppm) = 7,23-8,33 (7H, m, Aryl-H); 5,45 u. 4,23 (2H, AB-Syst. $J_{AB}$ = 13Hz, $CH_2$-7-Ring); 4,23 (2H, s, $CH_2$-C=O); 3,45-390 (8H, M Morpholin-H); 2,68 (3H, s, $CH_3$-Triazol).

Beispiel 5:

$^1H$-NMR ($CDCl_3$) δ (ppm) = 7,21-8,21 (7H, m, Aryl-H); 7,70 (1H, s, breit, NH); 5,49 u. 4,17 (2H, AB-System, JAB = 13Hz, $CH_2$-7-Ring); 4,98 (1H, m, Asp-CH) 3,79 u. 3,70 (6H, 2 s, $OCH_3$); 3,05 (2H, m, Asp-$CH_2$CH); 2,64 (3H, s, $CH_3$-Triazol).

Beispiel 6:

$^1H$-NMR ($CDCl_3$) δ (ppm) = 7,16-8,32 (7H, m, Aryl-H); 7,89 (1H, t, breit, NH) 7,59 (1H, s, breit, NH); 5,48 u. 4,13 (2H, AB-Syst. $J_{AB}$ = 13Hz, $CH_2$-7-Ring); 3,97-430 (4H, m, 2 × $CH_2$-C=O); 3,26-3,85 (8H, M, Morpholin-H); 2,62 (3H, s, $CH_3$-Triazol).

Beispiel 11:

$^1H$-NMR ($CDCl_3$) δ (ppm) = 7,14-8,26 (7H, m, Aryl-H); 7,78 (1H, s, breit, NH); 5,49 u. 4,09 (2H, AB-System, $J_{AB}$=13Hz, $CH_2$-7-Ring); 4,71 (1H, m, CH, Thiophen-Ring); 1,95-3,62 (4H, m, $CH_2CH_2$-Homothioserinlacton); 2,59 (3H, s, $CH_3$-Triazol).

Beispiel 19:

$^1H$-NMR ($CDCl_3$) δ (ppm) = 7,26-8,44 (8H, m, Aryl-H); 8,30 (1H, s, breit, NH); 5,41 u. 4,13 (2H, AB-Syst. $J_{AB}$ = 13Hz, $CH_2$-7-Ring); 4,21 (2H, s, Gly-$CH_2$); 3,40-3,89 (8H, m, Morpholin-H); 2,70 (3H, s, $CH_3$-Triazol).

Beispiel 21:

$^1H$-NMR ($CD_3OD$) δ (ppm) = 7,47-8,40 (7H, M. Aryl-H); 5,60 (2H, s, $CH_2$-7-Ring); 4,41 (1H, s, CH-NH); 4,23 (2H, s, Gly-$CH_2$); 3,47 - 3,91 (8H, m, Morpholin-H); 2,77 (3H, s, $CH_3$-Triazol).

Beispiel 25:

$^1H$-NMR ($CDCl_3$) δ (ppm) = 7,17-8,16 (7H, m, Aryl-H); 5,55 u. 4,15 (2H, AB-Syst., $J_{AB}$ = 13Hz,$CH_2$-7-Ring-); 7,59 (1H, s breit, NH), 5,33 (1H, m, CH); 3,45 - 394 (8H, m, Morpholin-H); 3,16 (2H, t, J = 6,5Hz, $SO_2CH_2$), 2,94 (3H, s, $SO_2CH_3$); 2,64 (3H, s, $CH_3$-Triazol); 2,32 (2H, m, $CH_2$-CH).

Beispiel 27:

$^1H$-NMR ($CDCl_3$) δ (ppm) = 7,02-8,20 (7H, M, Aryl-H); 7,16 (1H, d, J=8Hz,$C_6H_5$-CO-NH); 6,01 (1H, d, J=8Hz, -CO-NHiPr); 5,51 u. 4,13 (2H, AB-System, $J_{AB}$=13Hz, $CH_2$-7-Ring); 3,80-4,47 (2H, m NH-CH, Val-α-CH); 2,62 (3H, s, $CH_3$-Triazol); 2,09 (1H, m, CH-CH($CH_3$)$_2$); 1,12 u. 1,18 (6H, 2d, J=6Hz, ($CH_3$)$_2$-CH-C); 0,97 (6H, d, J=7Hz, ($CH_3$)$_2$-CH-N).

Beispiel 31:

$^1H$-NMR ($CDCl_3$) δ (ppm) = 7,09-7,61 (5H, m, Aryl-H; NH); 7,18 (1H, s, Thiophen-H); 5,08 (1H, m Leu-CH); 4,95 (2H, s, $CH_2$-7-Ring); 3,36-3,90 (8H, m, Morpholin-H); 2,75 (3H, s, $CH_3$-Triazol); 1,36-1,87 (3H, m, $CH_2$-CH); 0,93 u. 1,00 (6H, 2d, J=8Hz, ($CH_3$)$_2$-C);

Beispiel 35:

$^1H$-NMR ($CDCl_3$) δ (ppm) = 7,14-8,20 (8H m, Aryl-HNH); 5,52 u. 4,13 (2H AB-System, $J_{AB}$ =13 Hz, $CH_2$-7-Ring); 4,83 (1H, m, Ala-α-CH); 3,27-3,80 (5H, 2 × N-$CH_2$-, -CH-Cyclopropan); 1,43 (3H, d, J=6Hz, $CH_3$-CH); 0,88-2,28 (8H, m, $CH_2$-Pyrrolidin, $CH_2$-Cyclopropan).

Beispiel 38:

$^1H$-NMR ($CDCl_3$) δ (ppm) = 7,20-8,22, (7H, m, Aryl-H); 5,54 u. 4,14 (2H, AB-System, $J_{AB}$ =13Hz, $CH_2$-7-Ring); 5,23 (1H, dd, CH); 7,37 /1H, s, breit, NH); 3,50-3,92 (8H, m, Morpholin-H); 2,64 (3H, s, $CH_3$-Triazol); 2,57 (2H, t, J=7Hz, $SCH_2$); 2,07 (3H, s, $SCH_3$); 2,01 (2H, m, $CH_2$-CH).

Beispiel 42:

$^1$H-NMR ($CDCl_3$) δ (ppm) = 7,13-8,30 (7H, m, Aryl-H); 7,90 (1H, t, NH-$CH_2$-C=O); 6,7 (1H, t, breit, NH-$CH_2$-C=C), 5,49 u. 4,13 (2H, AB-Syst., $J_{AB}$ = 13Hz, $CH_2$-7-Ring-), 4,98 - 6,10 (3H, m, $CH_2$=CH-); 3,98 4,17 (2H, m, $CH_2$-C=); 3,89 (2H, t, J=6Hz, Gly-$CH_2$), 2,61 (3H, s, $CH_3$-Triazol).

Beispiel 44:

$^1$H-NMR ($CDCl_3$) δ (ppm) = 7,23-7,60 (4H, m, Aryl-H); 6,92 (1H, t, J = 3Hz, NH); 4,95 (2H, s, $CH_2$-7-Ring); 4,09 (2H, d, J=3Hz, Gly-$CH_2$); 3,80 (2H, s, $CH_2$-C=O);3,25-3,92 (8H, m, Morpholin-H); 2,74 (3H, s, $CH_3$-Triazol).

Beispiel 48:

$^1$H-NMR ($CDC1_3$) δ (ppm) = 7,20-7,61 (4H, m, Aryl-H); 6,66 (1H, t, J = 4Hz, NH); 6,44 (1H, s, Thiophen-H); 4,93 (2H, s, $CH_2$-7-Ring); 4,05 (2H, d, J = 4Hz, Gly-$CH_2$); 3,28-3,83 (8H, m, Morpholin-H); 3,15 (2H, t, J = 6,5Hz, $CH_2$-Thiophen); 2,61 (2H, t, J = 6,5Hz, $CH_2$-C=O); 2,70 (3H, s, $CH_3$-Triazol).

**Patentansprüche**

**1.** 1,4-Diazepine der allgemeinen Formel

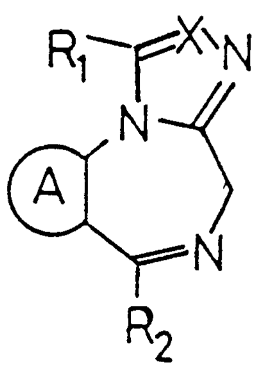

I

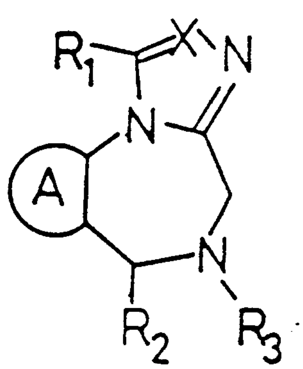

II

worin

- $R_1$ Wasserstoff, eine verzweigte oder unverzweigte Alkylgruppe mit 1 bis 4 Kohlenstoffatomen, die gegebenenfalls durch Hydroxy oder Halogen substituiert sein kann, eine Cyclopropylgruppe, eine Alkoxygruppe mit 1 bis 4 Kohlenstoffatomen, Halogen;
- $R_2$ Phenyl, wobei der Phenylring ein- oder mehrfach durch Methyl, Methoxy, Halogen, Nitro und/oder Trifluormethyl substituiert sein kann, α-Pyridyl;
- $R_3$ Wasserstoff oder eine verzweigte oder unverzweigte Alkylgruppe mit 1 bis 4 Kohlenstoffatomen;
- A einen ankondensierten Ring der allgemeinen Formel

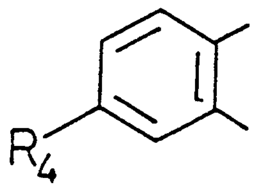

a

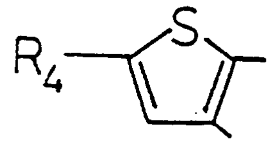

b

worin $R_4$ eine funktionelle Seitenkette der allgemeinen Formel

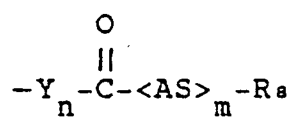

worin Y eine verzweigte oder unverzweigte Alkylgruppe mit n Kohlenstoffatomen,<AS> eine N-terminal angeknüpfte Aminosäure der allgemeinen Formel

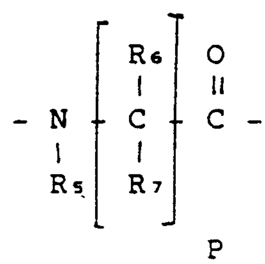

worin
$R_5$ Wasserstoff, eine verzweigte oder unverzweigte Alkylgruppe mit 1 bis 5 Kohlenstoffatomen;
$R_6$ und $R_7$, die gleich oder verschieden sein können, Wasserstoff, eine verzweigte oder unverzweigte Alkyl-, Alkenyl- oder Alkinylgruppe mit 1 bis 10 Kohlenstoffatomen, eine Cycloalkylgruppe mit 3 bis 6 Kohlenstoffatomen, eine Phenylgruppe, eine Benzylgruppe,
wobei $R_6$ und/oder $R_7$ im Fall einer $C_1$-$C_4$-Alkylgruppe gegebenenfalls ein- oder mehrfach substituiert ist, durch Hydroxy, $C_1$-$C_4$ Alkoxy, Cycloalkyl mit 3 bis 7 Kohlenstoffatomen, Halogen, eine Aminogruppe, die gegebenenfalls ein- oder zweifach durch verzweigtes oder unverzweigtes Alkyl, Alkoxycarbonyl oder Phenalkoxycarbonyl mit jeweils 1 bis 4 Kohlenstoffatomen in der Alkylkette substituiert sein kann, Guanidino, Ureido, Carboxy, $C_1$-$C_4$-Alkoxycarbonyl, Cyano, Carboxamid, $C_1$-$C_4$-Alkylcarbonyl, Mercapto, $C_1$-$C_4$-Alkylmercapto, Benzylmercapto, $C_1$-$C_4$-Alkylsulfoxyl, $C_1$-$C_4$ Alkylsulfonyl, 3-Indolyl, Imidazolyl, Pyrazolyl und/oder ein Säureamid der allgemeinen Formel

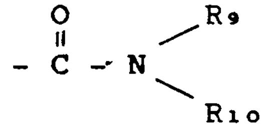

worin $R_9$ und $R_{10}$, die gleich oder verschieden sein können, Wasserstoff, eine verzweigte oder unverzweigte Alkyl-, Alkenyl- oder Alkinylgruppe mit 1 bis 4 Kohlenstoffatomen, wobei die Alkylgruppe durch eine Aminogruppe, die gegebenenfalls ein- oder zweifach durch verzweigtes oder unverzweigtes Alkyl, mit 1 bis 4 Kohlenstoffatomen in der Alkylkette oder

durch eine Hydroxygruppe substituiert sein kann;
oder
$R_9$ und $R_{10}$ gegebenenfalls einen 3- bis 6-gliedrigen Ring bilden, der als weitere Heteroatome Stickstoff, Sauerstoff oder Schwefel enthalten kann, wobei der Ring durch wenigstens eine verzweigte oder unverzweigte Alkylgruppe mit 1 bis 4 Kohlenstoffatomen, substituiert sein kann;
$R_6$ und/oder $R_7$ im Fall einer Phenyl oder einer Benzylgruppe, durch wenigstens eine Hydroxy-, Halogen-, Amino-, verzweigte- oder unverzweigte Alkylamino-, Dialkylamino- oder Alkoxygruppe mit 1 bis 4 Kohlenstoffatomen substituiert sein kann;
$R_8$ eine, gegebenenfalls durch eine Aminogruppe der allgemeinen Formel

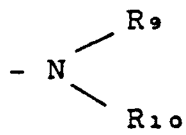

substituierte, verzweigte oder unverzweigte Alkoxy-oder Alkylthiogruppe mit 1 bis 4 Kohlenstoffatomen,
wobei $R_9$ und $R_{10}$ die zuvor genannte Bedeutung aufweisen,
oder eine Aminogruppe der allgemeinen Formel

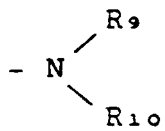

worin $R_9$ und $R_{10}$ die oben genannte Bedeutung haben;
$R_6$ und $R_7$ gegebenenfalls einen 3- bis 7-gliedrigen Ring bilden, der als weitere Heteroatome Stickstoff, Sauerstoff oder Schwefel enthalten kann und gegebenenfalls durch wenigstens eine verzweigte oder unverzweigte Alkylgruppe mit 1 bis 4 Kohlenstoffatomen substituiert sein kann;
oder
$R_5$ und $R_6$ oder $R_7$ gegebenenfalls einen 4- bis 7-gliedrigen Ring bilden, der gegebenenfalls als weitere Heteroatome Stickstoff, Sauerstoff oder Schwefel enthalten kann, wobei der Ring durch wenigstens eine verzweigte oder unverzweigte Alkylgruppe mit 1 bis 4 Kohlenstoffatomen substituiert sein kann;
oder
$R_6$ oder $R_7$ und $R_8$ gegebenenfalls einen 5- bis 7-gliedrigen Ring der allgemeinen Formel

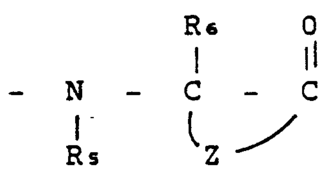

bilden,
wobei
Z eines der Heteroatome Stickstoff, Sauerstoff oder Schwefel bedeutet, wobei der Ring durch eine verzweigte oder unverzweigte Alkylgruppe mit 1 - 4 Kohlenstoffatomen substituiert sein kann;

X CH, C-Halogen oder Stickstoff,
n eine der Zahlen 0, 1, 2, 3, 4, 5, 6, 7 oder 8,
p eine der Zahlen 1, 2, 3, 4, 5, 6, 7 oder 8 und

m eine der Zahlen 1, 2 oder 3, bevorzugt 1, bedeuten, deren optisch aktive Verbindungen oder deren Racemate sowie gegebenenfalls deren physiologisch unbedenkliche Säureadditionssalze.

**2.** Verbindungen der allgemeinen Formel I oder II nach Anspruch 1, dadurch gekennzeichnet, daß
$R_1$, $R_3$ und A die zuvor genannte Bedeutung aufweisen,
$R_2$ Phenyl, wobei der Phenylring, bevorzugt in 2-Stellung, durch Halogen, besonders bevorzugt Chlor, substituiert sein kann,
$R_4$ eine funktionelle Seitenkette der allgemeinen Formel

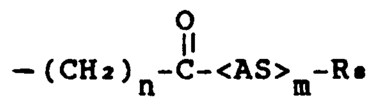

worin
<AS> eine Aminosäure der allgemeinen Formel

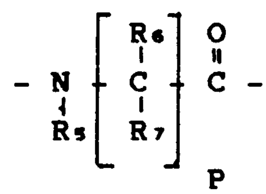

worin
$R_5$, $R_6$ und $R_7$ wie zuvor definiert sind,
$R_8$ die oben angegebene Bedeutung aufweist,
X CH oder Stickstoff,
n eine der Zahlen 0, 1, 2, 3, 4, 5, 6, 7 oder 8
p die Zahlen 1 oder 2 und
m die Zahlen 1, 2 oder 3, bevorzugt 1, bedeuten,
deren optisch aktive Verbindungen oder deren Racemate sowie gegebenenfalls deren physiologisch unbedenkliche Säureadditionssalze.

**3.** Verbindungen der allgemeinen Formel I oder II nach Anspruch 1 oder 2, dadurch gekennzeichnet, daß
$R_1$ Wasserstoff, Methyl, Ethyl, Cyclopropyl, Methoxy, Ethoxy oder Halogen, bevorzugt Chlor oder Brom,
$R_2$ Phenyl, wobei der Phenylring in 2-Stellung durch Halogen, bevorzugt Chlor, substituiert sein kann,
$R_3$ Wasserstoff oder Methyl, bevorzugt Wasserstoff,
A wie oben angegeben,
$R_4$ eine funktionelle Seitenkette der allgemeinen Formel

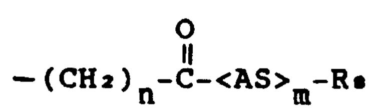

worin
<AS>eine Aminosäure der allgemeinen Formel

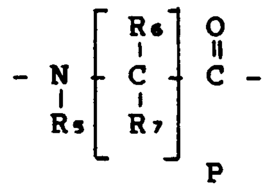

worin
$R_5$, $R_6$ und $R_7$ wie zuvor definiert sind,
$R_8$ wie oben angegeben ist,

X CH oder Stickstoff,

n im Fall von A = a eine der Zahlen 0 oder 1 und im Fall von A = b eine der Zahlen 0, 1, 2, 3, 4, 5, 6, 7 oder 8,

p eine der Zahlen 1 oder 2 und

m eine der Zahlen 1 oder 2, bevorzugt 1,

bedeuten, deren optisch aktive Verbindungen oder deren Racemate sowie deren physiologisch unbedenklichen Säureadditionssalze.

**4.** Verbindungen der allgemeinen Formel I oder II nach einem der Ansprüche 1 bis 3, dadurch gekennzeichnet, daß

$R_1$ Wasserstoff, Ethyl, Cyclopropyl, Ethoxy, Chlor oder Brom, bevorzugt Methyl oder Methoxy,

$R_2$ Phenyl, wobei der Phenylring in 2-Stellung durch Halogen, bevorzugt Chlor, substituiert sein kann,

$R_3$ Wasserstoff,

A wie oben angegeben,

$R_4$ eine funktionelle Seitenkette der allgemeinen Formel

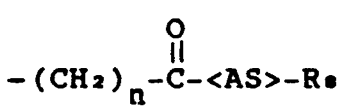

worin
<AS>eine $\alpha$-Aminosäure der allgemeinen Formel

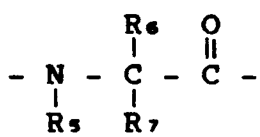

worin
$R_5$ Wasserstoff,
$R_6$ und $R_7$, die gleich oder verschieden sein können, Wasserstoff, eine verzweigte oder unverzweigte Alkylgruppe mit 1 bis 8, bevorzugt 1 bis 5 Kohlenstoffatomen, gegebenenfalls substituiert durch Alkoxycarbonyl, Alkylthio oder Alkylsulfonyl mit 1 bis 4 Kohlenstoffatomen in der Alkylkette,

$R_8$ Alkoxy mit 1 bis 4 Kohlenstoffatomen, bevorzugt Methoxy, Ethoxy oder bevorzugt eine Aminogruppe der allgemeinen Formel

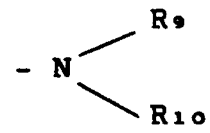

worin

$R_9$ und $R_{10}$, die gleich oder verschieden sein können, eine verzweigte oder unverzweigte Alkyl- oder Alkenylgruppe mit 1 bis 4 Kohlenstoffatomen oder $R_9$ und $R_{10}$ einen 5- oder 6-gliedrigen Ring bilden, der als weitere Heteroatome Stickstoff oder Sauerstoff enthalten kann, wobei der Ring durch wenigstens eine Alkylgruppe mit 1 bis 4 Kohlenstoffatomen, bevorzugt Methyl, substituiert sein kann,

X CH oder bevorzugt Stickstoff,

n im Fall von A = a eine der Zahlen 0 oder 1, besonders bevorzugt 0 und im Fall von A = b eine der Zahlen 0, 1, 2, 3 oder 4, bevorzugt 0, 1 oder 2,

bedeuten,

deren optisch aktive Verbindungen oder deren Racemate sowie gegebenenfalls deren physiologisch unbedenkliche Säureadditionssalze.

**5.** Verbindungen der allgemeinen Formel I oder II nach einem der Ansprüche 1 bis 4, dadurch gekennzeichnet, daß $R_4$ eine funktionelle Seitenkette der allgemeinen Formel

$$-\ Y_n\ -\ \overset{O}{\overset{\|}{C}}\ -\ \langle AS\rangle_m\ -\ R_8$$

worin AS wenigstens eine der folgenden Aminosäuren Aad, γ-Abu, ε-Aca, Ach, Acp, β-Aib, Δ-Ala, Ama, Apm, Apr, Arg, Asn, Asu, Cys, Gln, Glu, His, Ser, Hyl, Hyp, 3-Hyp, Ise, Lys, Nle, Nva, Pec, Phe, Phg, Pic, Pro, Tle, Pyr, Ser, Thr, Tyr, Trp, Ala, β-Ala, Gly, Val, Met, Asp, Sar, Met-($O_2$), Aib, Abu, Ile, oder Leu bedeutet,

$R_1$, $R_2$, $R_3$, A, $R_8$, X, n und m die zuvor genannte Bedeutung aufweisen, deren optische aktive Verbindungen oder deren Racemate sowie gegebenenfalls deren physiologisch unbedenkliche Säureadditionssalze.

**6.** Verfahren zur Herstellung von Verbindungen der allgemeinen Formel I oder II

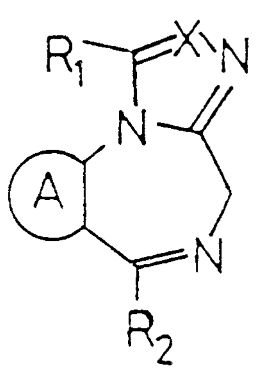

I

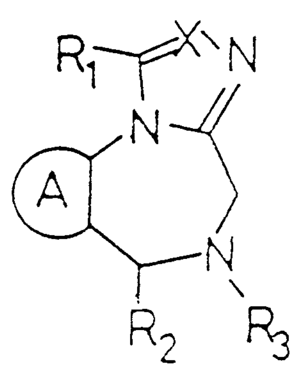

II

worin

$R_1$ Wasserstoff, eine verzweigte oder unverzweigte Alkylgruppe mit 1 bis 4 Kohlenstoffatomen, die gegebenenfalls durch Hydroxy oder Halogen substituiert sein kann, eine Cyclopropylgruppe, eine Alkoxygruppe mit 1 bis 4 Kohlenstoffatomen, Halogen;

$R_2$ Phenyl, wobei der Phenylring ein- oder mehrfach durch Methyl, Methoxy, Halogen, Nitro und/oder Trifluormethyl substituiert sein kann, α-Pyridyl;

$R_3$ Wasserstoff oder eine verzweigte oder unverzweigte Alkylgruppe mit 1 bis 4 Kohlenstoffatomen;

A einen ankondensierten Ring der allgemeinen Formel

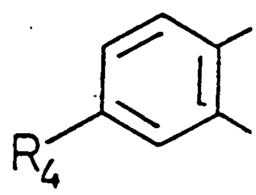

a

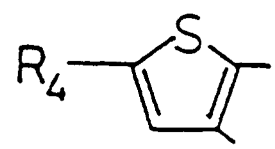

b

worin $R_4$ eine funktionelle Seitenkette der allgemeinen Formel

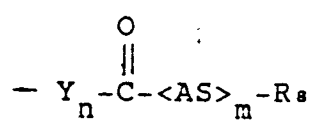

worin Y eine verzweigte oder unverzweigte Alkylgruppe mit n Kohlenstoffatomen,<AS> eine N-terminal angeknüpfte Aminosäure der allgemeinen Formel

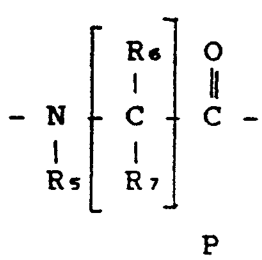

worin

$R_5$ Wasserstoff, eine verzweigte oder unverzweigte Alkylgruppe mit 1 bis 5 Kohlenstoffatomen;

$R_6$ und $R_7$, die gleich oder verschieden sein können, Wasserstoff, eine verzweigte oder unverzweigte Alkyl-, Alkenyl- oder Alkinylgruppe mit 1 bis 10 Kohlenstoffatomen, eine Cycloalkylgruppe mit 3 bis 6 Kohlenstoffatomen, eine Phenylgruppe, eine Benzylgruppe,

wobei $R_6$ und/oder $R_7$ im Fall einer Alkylgruppe gegebenenfalls ein- oder mehrfach substituiert ist durch Hydroxy, $C_1$-$C_4$-Alkoxy, Cycloalkyl mit 3 bis 7 Kohlenstoffatomen, Halogen, eine Aminogruppe, die gegebenenfalls ein- oder zweifach durch verzweigtes oder unverzweigtes Alkyl, Alkoxycarbonyl oder Phenalkoxycarbonyl mit jeweils 1 bis 4 Kohlenstoffatomen in der Alkylkette substituiert sein kann, Guanidino, Ureido, Carboxy, $C_1$-$C_4$-Alkoxycarbonyl, Cyano, Carboxamid, $C_1$-$C_4$-Alkylcarbonyl, Mercapto, $C_1$-$C_4$-Alkylmercapto, Benzylmercapto, $C_1$-$C_4$-Alkylsulfoxyl, $C_1$-$C_4$ Alkylsulfonyl, 3-Indolyl, Imidazolyl, Pyrazolyl und/oder ein Säureamid der allgemeinen Formel

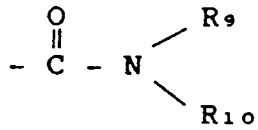

worin $R_9$ und $R_{10}$, die gleich oder verschieden sein können, Wasserstoff, eine verzweigte oder unverzweigte Alkyl-, Alkenyl- oder Alkinylgruppe mit 1 bis 4 Kohlenstoffatomen, wobei die Alkylgruppe durch eine Aminogruppe, die gegebenenfalls ein- oder zweifach durch verzweigtes oder unverzweigtes Alkyl, mit 1 bis 4 Kohlenstoffatomen in der Alkylkette oder durch eine Hydroxygruppe substituiert sein kann;
oder
$R_9$ und $R_{10}$ gegebenenfalls einen 3- bis 6-gliedrigen Ring bilden, der als weitere Heteroatome Stickstoff, Sauerstoff oder Schwefel enthalten kann, wobei der Ring durch wenigstens eine Verzweigte oder unverzweigte Alkylgruppe mit 1 bis 4 Kohlenstoffatomen, substituiert sein kann;
$R_6$ und/oder $R_7$ im Fall einer Phenyl- oder einer Benzylgruppe, durch wenigstens eine Hydroxy-, Halogen-, Amino-, verzweigte- oder unverzweigte Alkylamino-, Dialkylamino- oder Alkoxygruppe mit 1 bis 4 Kohlenstoffatomen substituiert sein kann;
$R_8$ eine, gegebenenfalls durch eine Aminogruppe der allgemeinen Formel

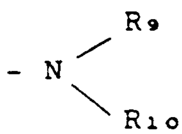

substituierte, verzweigte oder unverzweigte Alkoxy-oder Alkylthiogruppe mit 1 bis 4 Kohlenstoffatomen,
wobei $R_9$ und $R_{10}$ die zuvor genannte Bedeutung aufweisen,
oder eine Aminogruppe der allgemeinen Formel

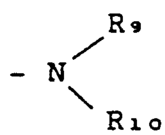

worin $R_9$ und $R_{10}$ die oben genannte Bedeutung haben;
$R_6$ und $R_7$ gegebenenfalls einen 3- bis 7-gliedrigen Ring bilden, der als weitere Heteroatome Stickstoff, Sauerstoff oder Schwefel enthalten kann und gegebenenfalls durch wenigstens eine verzweigte oder unverzweigte Alkylgruppe mit 1 bis 4 Kohlenstoffatomen substituiert sein kann;
oder
$R_5$ und $R_6$ oder $R_7$ gegebenenfalls einen 4- bis 7-gliedrigen Ring bilden, der gegebenenfalls als weitere Heteroatome Stickstoff, Sauerstoff oder Schwefel enthalten kann, wobei der Ring durch wenigstens eine verzweigte oder unverzweigte Alkylgruppe mit 1 bis 4 Kohlenstoffatomen substituiert sein kann;
oder
$R_6$ oder $R_7$ und $R_8$ gegebenenfalls einen 5- bis 7-gliedrigen Ring der allgemeinen Formel

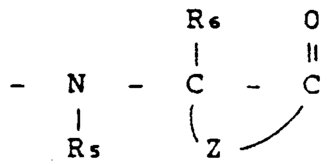

bilden,
wobei
Z eines der Heteroatome Stickstoff, Sauerstoff oder Schwefel bedeutet, wobei der Ring durch eine verzweigte oder unverzweigte Alkylgruppe mit 1 - 4 Kohlenstoffatomen substituiert sein kann;

X CH, C-Halogen oder Stickstoff,
n eine der Zahlen 0, 1, 2, 3, 4, 5, 6, 7 oder 8,
p eine der Zahlen 1, 2, 3, 4, 5, 6, 7 oder 8 und
m eine der Zahlen 1, 2 oder 3, bevorzugt 1, bedeuten, deren optisch aktive Verbindungen oder deren Racemate, dadurch gekennzeichnet, daß man eine Verbindung der allgemeinen Formel IIIa oder IIIb,

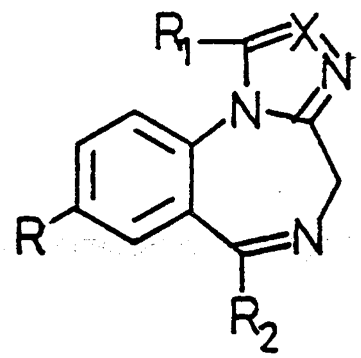

IIIa

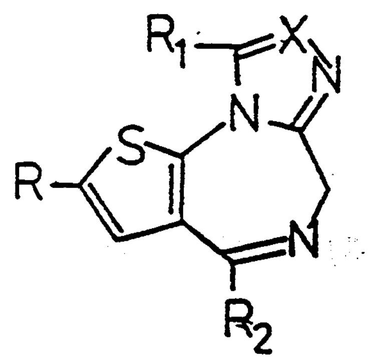

IIIb

worin die Reste $R_1$, $R_2$ und X die angegebene Bedeutung haben und R eine Seitenkette der allgemeinen Formel

$- Y_n - COOH$

ist, worin Y und n wie zuvor definiert sind,

a) mit einer Verbindung der allgemeinen Formel IV

$H - <AS>_m - R_8$

worin AS, $R_8$ und m die zuvor genannte Bedeutung aufweisen, in Gegenwart eines Carbodiimids oder

b) die Säure der allgemeinen Formel IIIa oder IIIb in ein reaktives Säurederivat überführt und anschließend mit einer Verbindung der allgemeinen Formel IV umsetzt,

gegebenenfalls anschließend eine Verbindung der allgemeinen Formel I durch Reduktion in eine Verbindung der allgemeinen Formel II überführt und gegebenenfalls eine Verbindung der allgemeinen Formel I oder II in ihre Säureadditionssalze überführt.

7. Verfahren zur Herstellung von Verbindungen der allgemeinen Formel I nach Anspruch 6, worin $R_1$, $R_2$,

$R_4$ und X wie zuvor definiert sind und $R_8$ eine Aminogruppe der allgemeinen Formel

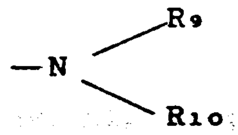

worin $R_9$ und $R_{10}$ wie vor definiert sind, dadurch gekennzeichnet, daß man eine Verbindung der allgemeinen Formel

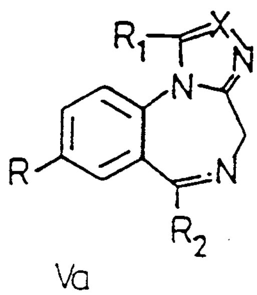

Va

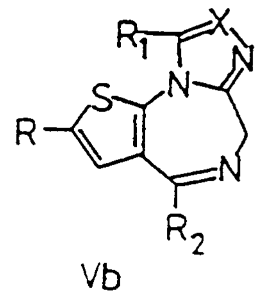

Vb

worin R = -$Y_n$-CO-<AS>$_m$-OH bedeutet
mit einem Amin der allgemeinen Formel

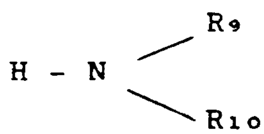

worin $R_9$ und $R_{10}$ wie oben definiert sind, nach an sich bekannten Verfahren umsetzt.

**8.** Pharmazeutische Präparate, enthaltend als Wirkstoffe Verbindungen der allgemeinen Formel I oder II in Kombination mit üblichen Hilfs- und/oder Trägerstoffen.

**9.** Verfahren zur Herstellung von pharmazeutischen Präparaten nach Anspruch 8, dadurch gekennzeichnet, daß man Verbindungen der allgemeinen Formel I oder II mit üblichen galenischen Hilfs- und/oder Trägerstoffen zu üblichen pharmazeutischen Anwendungsformen verarbeitet.

**10.** Verbindungen der allgemeinen Formel I oder II gemäß einem der Ansprüche 1 bis 5 zur Verwendung für die Herstellung von Arzneimitteln mit PAF-antagonistischer Wirkung.

**11.** Verwendung einer Verbindung der allgemeinen Formel I oder II zur Herstellung eines Arzneimittels zur Behandlung pathologischer Zustände und Krankheiten, an denen PAF (Plättchen Aktivierender Faktor) beteiligt ist.

**12.** Zwischenverbindungen der allgemeinen Formel

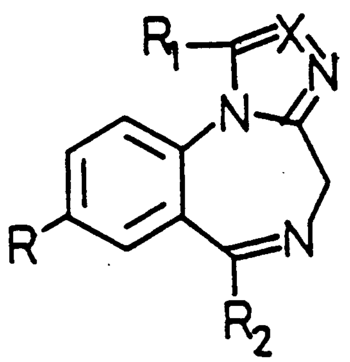

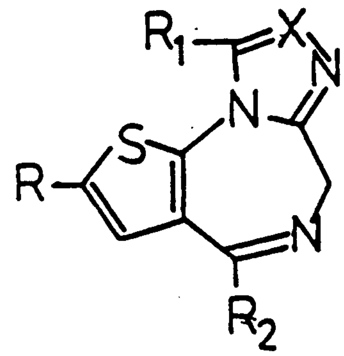

a

b

III / VI

worin R eine Seitenkette der allgemeinen Formel

$-Y_n-COOR'$

mit R' = Wasserstoff oder eine verzweigte oder unverzweigte Alkylgruppe mit I bis 4 Kohlenstoffatomen bedeutet
und $R_1$, $R_2$, X, Y und n die in den Ansprüchen I bis 5 genannte Bedeutung aufweisen können.

**13.** Zwischenverbindungen der allgemeinen Formel

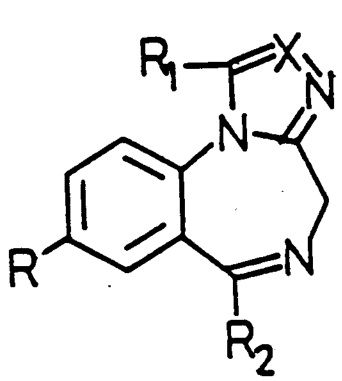

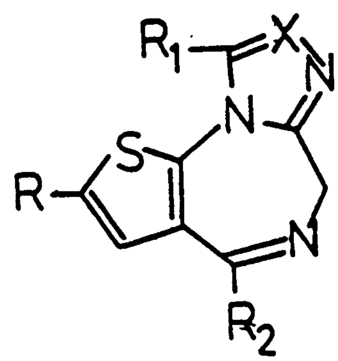

a

b

V

worin R eine Seitenkette der allgemeinen Formel

$-Y_n$ - CO - <AS>$_m$ - OH

und $R_1$, $R_2$, X, Y, n, m und <AS> die in den Ansprüchen 1 bis 5 genannte Bedeutung aufweisen können.

## Claims

**1.** 1,4-Diazepines of general formula

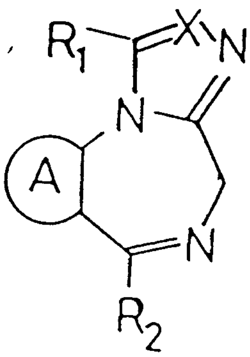

I

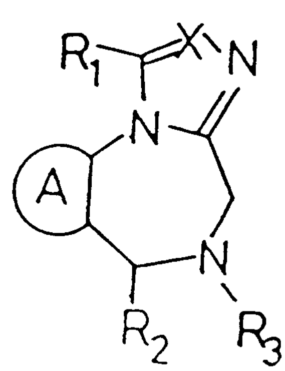

II

in which

- $R_1$ denotes hydrogen, a branched or unbranched alkyl group having 1 to 4 carbon atoms (and can optionally be substituted by hydroxyl or halogen), a cyclopropyl group, an alkoxy group having 1 to 4 carbon atoms, or halogen;
- $R_2$ denotes phenyl, it being possible for the phenyl ring to be substituted, once or several times by methyl, methoxy, halogen, nitro and/or trifluoromethyl, or denotes $\alpha$-pyridyl;
- $R_3$ denotes hydrogen or a branched or unbranched alkyl group having 1 to 4 carbon atoms;
- A denotes a fused-on ring of the general formula

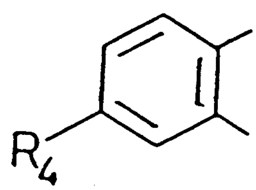

a

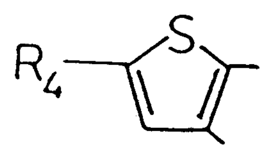

b

in which $R_4$ denotes a functional side-chain of the general formula

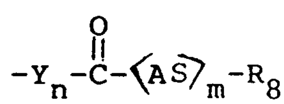

in which Y denotes a branched or unbranched alkyl group with n carbon atoms, <AS> denotes amino acid linked N-terminally, of the general formula

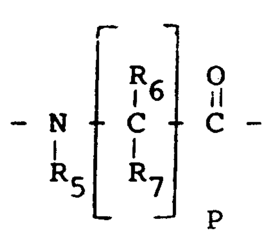

in which
$R_5$ denotes hydrogen, a branched or unbranched alkyl group having 1 to 5 carbon atoms;
$R_6$ and $R_7$, which can be identical or different, denote hydrogen, a branched or unbranched alkyl, alkenyl or alkynyl group having 1 to 10 carbon atoms, a cycloalkyl group having 3 to 6 carbon atoms, a phenyl group, a benzyl group (it being possible when $R_6$ and/or $R_7$ denotes a $C_{1-4}$-alkyl group, for $R_6$ and/or $R_7$ to be optionally mono- or poly-substituted by hydroxyl, $C_{1-4}$-alkoxy, cycloalkyl having 3-7 carbon atoms,
halogen, an amino group, (which can optionally be mono- or di-substituted by branched or unbranched alkyl, alkoxycarbonyl or phenalkoxycarbonyl, in each case having 1 to 4 carbon atoms in the alkyl chain),
guanidino, ureido, carboxy, $C_{1-4}$-alkoxycarbonyl, cyano, carboxamide, $C_{1-4}$-alkylcarbonyl, mercapto, $C_{1-4}$-alkylmercapto, benzylmercapto, $C_{1-4}$-alkylsulphoxyl, $C_{1-4}$-alkylsulfonyl, 3-indolyl, imidazolyl, pyrazolyl and/or an acid amide of the general formula

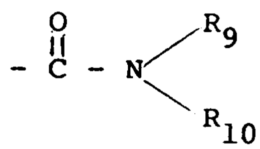

in which $R_9$ and $R_{10}$, which can be identical or different, denote hydrogen, a branched or unbranched alkyl, alkenyl or alkynyl group having 1 to 4 carbon atoms, it being possible for the alkyl group to be substituted by an amino group which can optionally be substituted once or twice by branched or unbranched alkyl having 1 to 4 carbon atoms in the alkyl chain, or by one hydroxyl group, or
$R_9$ and $R_{10}$ optionally form a 3- to 6-membered ring which can contain as further heteroatoms nitrogen, oxygen or sulphur, it being possible for the ring to be substituted by at least one branched or unbranched alkyl group having 1 to 4 carbon atoms);
$R_6$ and/or $R_7$ in the case of a phenyl or a benzyl group, can be substituted by at least one hydroxyl, halogen, amino, branched or unbranched alkylamino, dialkylamino or alkoxy group having 1 to 4 carbon atoms;
$R_8$ denotes a branched or unbranched alkoxy or alkylthio group which has 1 to 4 carbon atoms and is optionally substituted by an amino group of the general formula

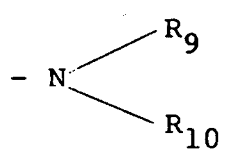

$R_9$ and $R_{10}$ having the above-mentioned meaning, or denotes an amino group of the general formula

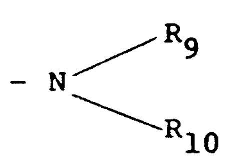

in which $R_9$ and $R_{10}$ have the above-mentioned meaning;
$R_6$ and $R_7$ optionally form a 3- to 7-membered ring which can contain as further hetero atoms nitrogen, oxygen or sulphur and can optionally be substituted by at least one branched or unbranched alkyl group having 1 to 4 carbon atoms; or

$R_5$ and $R_6$ or $R_7$ optionally form a 4- to 7-membered ring which can optionally contain as further heteroatoms nitrogen, oxygen or sulphur, it being possible for the ring to be substituted by at least one branched or unbranched alkyl group having 1 to 4 carbon atoms;
or
$R_6$ or $R_7$ and $R_8$ optionally form a 5- to 7-membered ring of the general formula

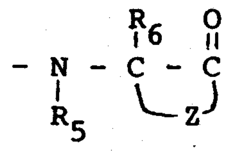

in which
Z denotes one of the heteroatoms, nitrogen, oxygen or sulphur, it being possible for the ring to be substituted by a branched or unbranched alkyl group having 1-4 carbon atoms;

X denotes CH, C-halogen or nitrogen,
n denotes one of the numbers 0, 1, 2, 3, 4, 5, 6, 7 or 8,
p denotes one of the numbers, 1, 2, 3, 4, 5, 6, 7 or 8 and
m denotes one of the numbers 1, 2 or 3, preferably 1, their optically active isomers or their racemates and, where appropriate, their physiologically acceptable acid addition salts.

**2.** Compounds of the general formula I or II according to claim 1, characterised in that
$R_1$, $R_3$ and A have the above-mentioned meaning,
$R_2$ denotes phenyl, it being possible for the phenyl ring to be substituted, preferably in the 2-position by halogen, particularly preferably chlorine,
$R_4$ denotes a functional side-chain of the general formula

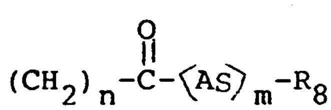

in which AS denotes an amino acid of the general formula

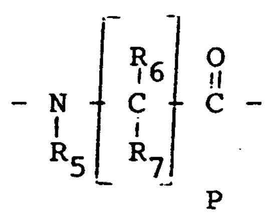

in which
$R_5$, $R_6$ and $R_7$ are as defined above,
$R_8$ has the above-mentioned meaning,
X denotes CH or nitrogen,
n denotes one of the numbers 0, 1, 2, 3, 4, 5, 6, 7 or 8,
p denotes the numbers, 1 or 2, and
m denotes the numbers 1, 2 or 3, preferably 1, and their optically active isomers or their racemates, and, where appropriate, their physiologically acceptable acid addition salts.

**3.** Compounds of the general formula I or II according to claim 1 or 2, characterised in that
$R_1$ denotes hydrogen, methyl, ethyl, cyclopropyl, methoxy, ethoxy or halogen, preferably chlorine or bromine,

$R_2$ denotes phenyl, it being possible for the phenyl ring to be substituted in the 2-position by halogen, preferably chlorine,

$R_3$ denotes hydrogen or methyl, preferably hydrogen,

A is as indicated above,

$R_4$ denotes a functional side-chain of the general formula

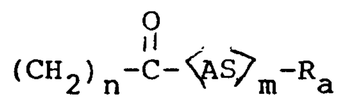

in which <AS> denotes an amino acid of the general formula

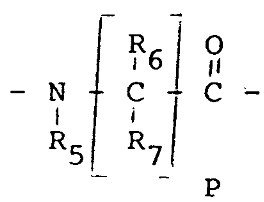

in which

$R_5$, $R_6$ and $R_7$ are as defined above,

$R_8$ is as indicated above,

X denotes CH or nitrogen,

n in the case of A = a denotes one of the numbers 0 or 1, and in the case of A = b denotes one of the numbers 0, 1, 2, 3, 4, 5, 6, 7 or 8,

p denotes one of the numbers, 1 or 2, and

m denotes the numbers 1 or 2, preferably 1, and

their optically active isomers or their racemates, and, their physiologically acceptable acid addition salts.

**4.** Compounds of the general formula I or II according to one of claims 1 to 3, characterised in that

$R_1$ denotes hydrogen; ethyl, cyclopropyl, ethoxy chlorine or bromine, preferably methyl or methoxy,

$R_2$ denotes phenyl, it being possible for the phenyl ring to be substituted in the 2-position by halogen, preferably chlorine,

$R_3$ denotes hydrogen,

A is as indicated above,

$R_4$ denotes a functional side-chain of the general formula

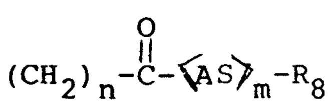

in which <AS> denotes an α-amino acid of the general formula

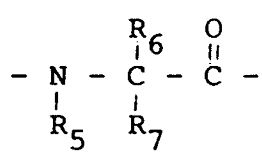

in which
$R_5$ denotes hydrogen,
$R_6$ and $R_7$, which can be identical or different, denote hydrogen, a branched or unbranched alkyl group which has 1 to 8, preferably 1 to 5 carbon atoms and is optionally substituted by alkoxycarbonyl, alkylthio or alkylsulfonyl with 1 to 4 carbon atoms in the alkyl chain,

$R_8$ denotes alkoxy having 1 to 4 carbon atoms, preferably methoxy or ethoxy, or preferably an amino group of the general formula

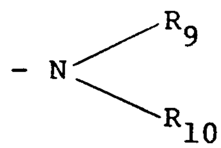

in which $R_9$ and $R_{10}$, which can be identical or different, denote a branched or unbranched alkyl or alkenyl group having 1 to 4 carbon atoms, or
$R_9$ and $R_{10}$ form a 5- or 6-membered ring which can contain as further heteroatoms nitrogen or oxygen, it being possible for the ring to be substituted by at least one alkyl group having 1 to 4 carbon atoms, preferably methyl,

X denotes CH or, preferably nitrogen,

n in the case of A = a denotes one of the numbers 0 or 1, particularly preferably 0, and in the case of A = b denotes one of the numbers 0, 1, 2, 3 or 4, preferably 0, 1 or 2,

their optically active isomers or their racemates, and, where appropriate, their physiologically acceptable acid addition salts.

**5.** Compounds of the general formula I or II according to one of claims 1 to 4, characterised in that $R_4$ denotes a functional side-chain of the general formula

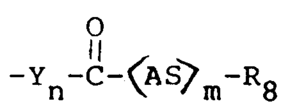

in which AS denotes at least one of the following amino acids
Aad, $\gamma$-Abu, $\epsilon$-Aca, Ach, Acp, $\beta$-Aib, $\Delta$-Ala, Ama, Apm, Apr, Arg, Asn, Asu, Cys, Gln, Glu, His, Ser, Hyl, Hyp, 3-Hyp, Ise, Lys, Nle, Nva, Pec, Phe, Phg, Pic, Pro, Tle, Pyr, Ser, Thr, Tyr, Trp, Ala, $\beta$-Ala, Gly, Val, Met, Asp, Sar, Met-($O_2$), Aib, Abu, Ile, or Leu,
$R_1$, $R_2$, $R_3$, A $R_8$, X , n and m have the above-mentioned meaning, their optically active isomers or their racemates and, where appropriate, their physiologically acceptable acid addition salts.

**6.** Process for the preparation of compounds of the general formula I or II

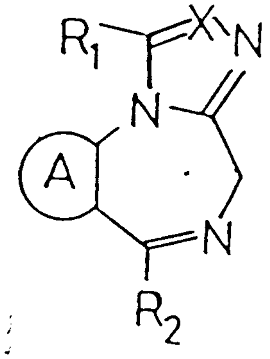

I

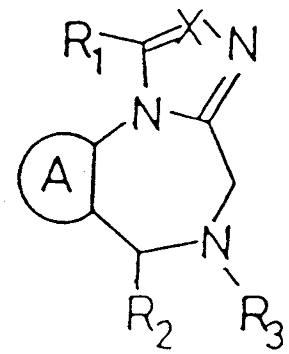

II

in which

R₁ denotes hydrogen, a branched or unbranched alkyl group having 1 to 4 carbon atoms (and can optionally be substituted by hydroxyl or halogen), a cyclopropyl group, an alkoxy group having 1 to 4 carbon atoms, or halogen;

$R_2$ denotes phenyl, it being possible for the phenyl ring to be substituted, once or several times by methyl, methoxy, halogen, nitro and/or trifluoromethyl, or denotes $\alpha$-pyridyl;

$R_3$ denotes hydrogen or a branched or unbranched alkyl group having 1 to 4 carbon atoms;

A denotes a fused-on ring of the general formula

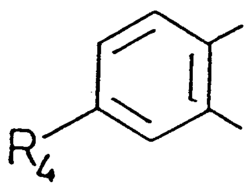

a

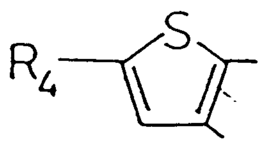

b

in which $R_4$ denotes a functional side-chain of the general formula

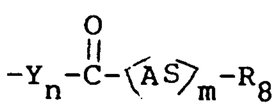

in which Y denotes a branched or unbranched alkyl group with n carbon atoms, <AS> denotes amino acid linked N-terminally, of the general

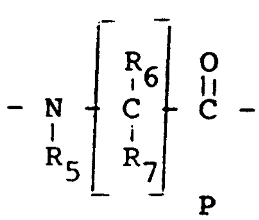

in which

$R_5$ denotes hydrogen, a branched or unbranched alkyl group having 1 to 5 carbon atoms;

$R_6$ and $R_7$, which can be identical or different, denote hydrogen, a branched or unbranched alkyl, alkenyl or alkynyl group having 1 to 10 carbon atoms, a cycloalkyl group having 3 to 6 carbon atoms, a phenyl group, a benzyl group (it being possible when $R_6$ and/or $R_7$ denotes an alkyl group for $R_6$ and/or $R_7$ to be optionally mono- or polysubstituted by hydroxyl, $C_{1-4}$-alkoxy, cycloalkyl having 3-7 carbon atoms, halogen, an amino group (which can optionally be mono- or di-substituted by branched or unbranched alkyl, alkoxycarbonyl or phenalkoxycarbonyl, in each case having 1 to 4 carbon atoms in the alkyl chain),

guanidino, ureido, carboxy, $C_{1-4}$-alkoxycarbonyl, cyano, carboxamide, $C_{1-4}$-alkylcarbonyl, mercapto, $C_{1-4}$-alkylmercapto, benzylmercapto, $C_{1-4}$-alkylsulfoxyl, $C_{1-4}$-alkylsulfonyl, 3-indolyl, imidazolyl, pyrazolyl and/or an acid amide of the general formula

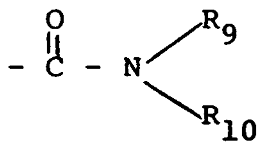

in which $R_9$ and $R_{10}$, which can be identical or different, denote hydrogen, a branched or unbranched alkyl, alkenyl or alkynyl group having 1 to 4 carbon atoms, it being possible for the alkyl group to be substituted by an amino group which can optionally be substituted once or twice by branched or unbranched alkyl having 1 to 4 carbon atoms in the alkyl chain, or by one hydroxyl group, or

$R_9$ and $R_{10}$ optionally form a 3- to 6-membered ring which can contain as further heteroatoms nitrogen, oxygen or sulphur, it being possible for the ring to be substituted by at least one branched or unbranched alkyl group having 1 to 4 carbon atoms);

$R_6$ and/or $R_7$ in the case of a phenyl or a benzyl group, can be substituted by at least one hydroxyl, halogen, amino, branched or unbranched alkylamino, dialkylamino or alkoxy group having 1 to 4 carbon atoms;

$R_8$ denotes a branched or unbranched alkoxy or alkylthio group which has 1 to 4 carbon atoms and is optionally substituted by an amino group of the general formula

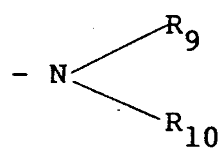

$R_9$ and $R_{10}$ having the above-mentioned meaning, or denotes an amino group of the general formula

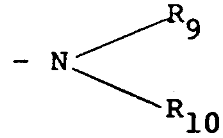

in which $R_9$ and $R_{10}$ have the above-mentioned meaning;

$R_6$ and $R_7$ optionally form a 3- to 7-membered ring which can contain as further hetero atoms nitrogen, oxygen or sulphur and can optionally be substituted by at least one branched or unbranched alkyl group having 1 to 4 carbon atoms; or

$R_5$ and $R_6$ or $R_7$ optionally form a 4- to 7-membered ring which can optionally contain as further heteroatoms nitrogen, oxygen or sulphur, it being possible for the ring to be substituted by at least one branched or unbranched alkyl group having 1 to 4 carbon atoms;

or
$R_6$ or $R_7$ and $R_8$ optionally form a 5- to 7-membered ring of the general formula

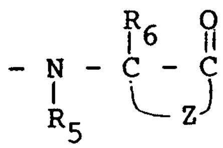

in which
Z denotes one of the heteroatoms, nitrogen, oxygen or sulphur, it being possible for the ring to be substituted by a branched or unbranched alkyl group having 1-4 carbon atoms;

X denotes CH, C-halogen or nitrogen,

n denotes one of the numbers 0, 1, 2, 3, 4, 5, 6, 7 or 8,

p denotes one of the numbers, 1, 2, 3, 4, 5, 6, 7 or 8 and

m denotes one of the numbers 1, 2 or 3, preferably 1, their optically active isomers or their racemates and, where appropriate, their physiologically acceptable acid addition salts, characterised in that a compound of the general formula IIIa or IIIb

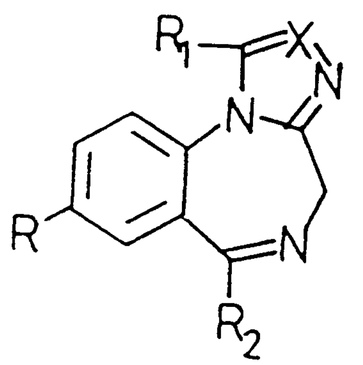

IIIa

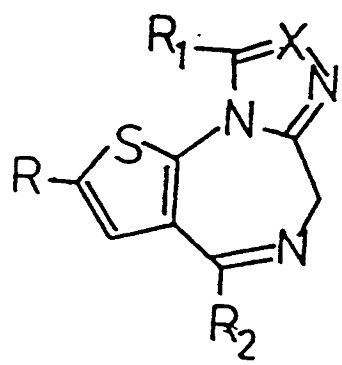

IIIb

in which
the radicals $R_1$, $R_2$ and X have the indicated meaning, and R is a side-chain of the general formula

$-Y_n-COOH$

in which Y and n are as defined previously,
a) is reacted with a compound of the general formula IV

$H-$<AS>$_m-R_8$

in which AS , $R_8$ and m have the above-mentioned meaning, in the presence of a carbodiimide, or
b) the acid of the general formula IIIa or IIIb is converted into a reactive acid derivative which is then reacted with a compound of the general formula IV,
and, where appropriate, a compound of the general formula I is subsequently converted by reduction into a compound of the general formula II, and, where appropriate, a compound of the general formula II is converted into its acid addition salts.

**7.** Process for the preparation of compounds of the general formula I according to claim 6, in which $R_1$, $R_2$, $R_4$ and X are as defined before, and $R_8$ is an amino group of the general formula

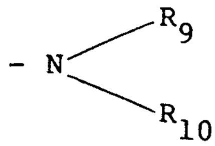

in which $R_9$ and $R_{10}$ are as defined before, characterised in that a compound of the general formula

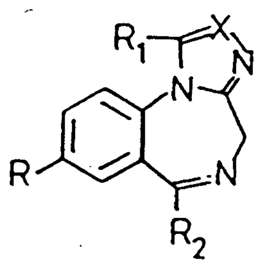

Va

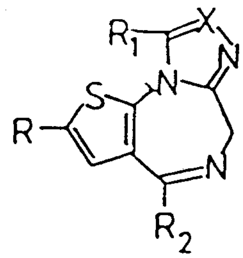

Vb

in which R denotes $-Y_n-CO-<AS>_m-OH$ is reacted, by processes, known per se, with an amine of the general formula

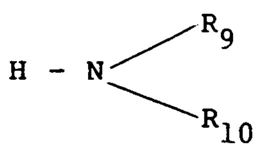

in which $R_9$ and $R_{10}$ are as defined above.

**8.** Pharmaceutical products containing as active ingredients compounds of the general formula I or II in combination with customary auxilaries and/or vehicles.

**9.** Process for the preparation of pharmaceutical products according to claim 8, characterised in that compounds of the general formula I or II are processed together with customary formulating auxiliaries and/or vehicles to give customary pharmaceutical presentations.

**10.** Compounds of the general formula I or II according to claims 1 to 5, for use for the preparation of medicaments having a PAF-antagonistic action.

**11.** Use of a compound of general formula I or II for preparing a pharmaceutical composition for the treatment of pathological states and diseases in which PAF (platelet activating factor) is concerned.

**12.** Intermediate compounds of general formula

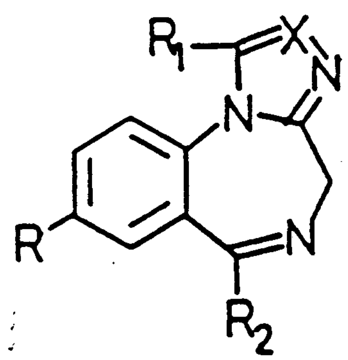

a

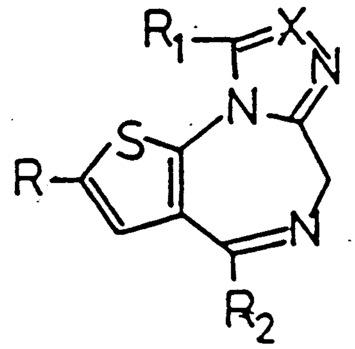

b

III / VI

wherein R denotes a side chain of general formula

-$Y_n$-COOR'

where R' = hydrogen or a branched or unbranched $C_{1-4}$-alkyl group and $R_1$, $R_2$, X, Y and n may have the meanings given in claims 1 to 5.

**13.** Intermediate compounds of general formula

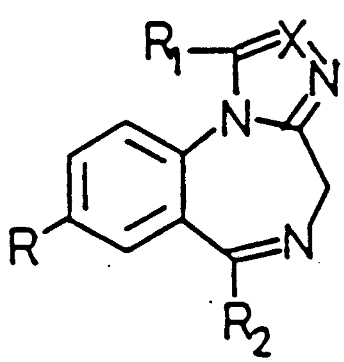

a

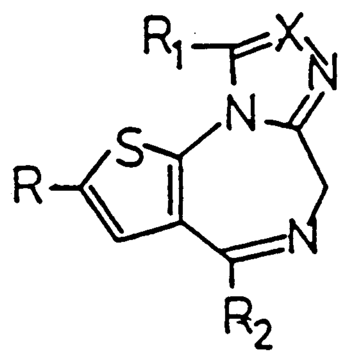

b

V

wherein R denotes a side chain of general formula

-$Y_n$ - CO - <AS>$_m$ - OH

and $R_1$, $R_2$, X, Y, n, m and <AS> may have the meanings given in claims 1 to 5.

**Revendications**

**1.** 1,4-diazépines de formule générale

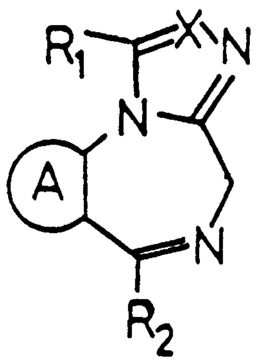

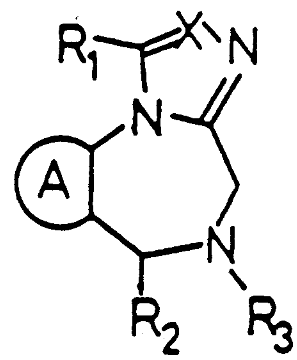

I

II

dans laquelle $R_1$ représente l'hydrogène, un groupe alkyle linéaire ou ramifié de 1 à 4 atomes de carbone qui peut éventuellement être substitué par un hydroxy ou un halogène, un groupe cyclopropyle, un groupe alcoxy de 1 à 4 atomes de carbone, un halogène;
$R_2$ représente un phényle, le cycle phényle pouvant être substitué une ou plusieurs fois par un méthyle, un méthoxy, un halogène, un nitro et/ou trifluorométhyle; un $\alpha$-pyridyle;
$R_3$ représente l'hydrogène ou un groupe alkyle linéaire ou ramifié de 1 à 4 atomes de carbone;
A représente un cycle condensé de formule générale

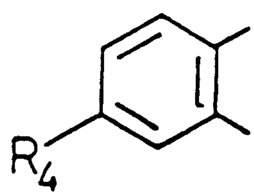

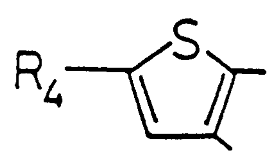

a

b

dans laquelle $R_4$ représente une chaîne latérale fonctionnelle de formule générale

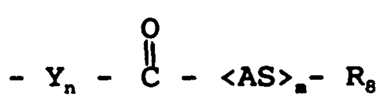

dans laquelle Y représente un groupe alkyle linéaire ou ramifié de n atomes de carbone, <AS> est un aminoacide relié par le N terminal de formule générale

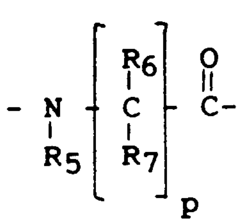

dans laquelle $R_5$ représente l'hydrogène, un groupe alkyle linéaire ou ramifié de 1 à 5 atomes de carbone;

$R_6$ et $R_7$, qui peuvent être identiques ou différents, représentent l'hydrogène, un groupe alkyle, alcényle ou alcynyle linéaire ou ramifié de 1 à 10 atomes de carbone, un groupe cycloalkyle de 3 à 6 atomes de carbone, un groupe phényle, un groupe benzyle,

$R_6$ et/ou $R_7$, dans le cas d'un groupe alkyle $C_1$-$C_4$, pouvant éventuellement être substitués une ou plusieurs fois par un hydroxy, un alcoxy en $C_1$-$C_4$, un cycloalkyle de 3 à 7 atomes de carbone, un halogène, un groupe amino qui peut éventuellement être substitué une ou deux fois par un alkyle, un alcoxycarbonyle ou un phénylalcoxycarbonyle, linéaires ou ramifiés, et contenant chacun 1 à 4 atomes de carbone dans la chaîne alkyle, un guanidino, un uréido, un carboxy, un alcoxycarbonyle en $C_1$-$C_4$, un cyano, un carboxamide, un alkylcarbonyle en $C_1$-$C_4$, un mercapto, un alkylmercapto en $C_1$-$C_4$, un benzylmercapto, un alkylsulfoxyle en $C_1$-$C_4$, un alkylsulfonyle en $C_1$-$C_4$, un 3-indolyle, un imidazolyle, un pyrazolyle et/ou un amide d'acide de formule générale

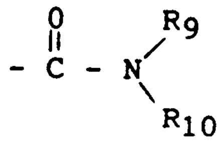

dans laquelle $R_9$ et $R_{10}$, qui peuvent être identiques ou différents, représentent l'hydrogène, un groupe alkyle, alcényle ou alcynyle linéaire ou ramifié de 1 à 4 atomes de carbone, le groupe alkyle pouvant être substitué par un groupe amino qui peut éventuellement être substitué une ou deux fois par un groupe alkyle linéaire ou ramifié de 1 à 4 atomes de carbone dans la chaîne alkyle, ou par un groupe hydroxy;

ou bien

$R_9$ et $R_{10}$ forment éventuellement un cycle de 3 à 6 chaînons qui peut contenir comme autres hétéroatomes de l'azote, de l'oxygène ou du soufre, le cycle pouvant être substitué par au moins un groupe alkyle linéaire ou ramifié de 1 à 4 atomes de carbone;

$R_6$ et/ou $R_7$, quand ils représentent un groupe phényle ou benzyle, peuvent être substitués par au moins un groupe hydroxy, halogène, amino ou un groupe alkylamino, dialkylamino ou alcoxy linéaire ou ramifié de 1 à 4 atomes de carbone;

$R_8$ représente un groupe alcoxy ou alkylthio linéaire ou ramifié de 1 à 4 atomes de carbone éventuellement substitué par un groupe amino de formule générale

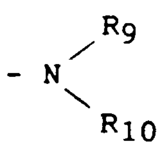

dans laquelle $R_9$ et $R_{10}$ ont la signification donnée ci-dessus, ou un groupe amino de formule générale

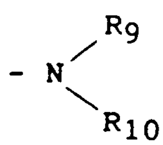

dans laquelle $R_9$ et $R_{10}$ ont la signification donnée ci-dessus;

$R_6$ et $R_7$ forment éventuellement un cycle de 3 à 7 chaînons qui peut contenir comme autres hétéroatomes de l'azote, de l'oxygène ou du soufre et qui peut éventuellement être substitué par au moins un groupe alkyle linéaire ou ramifié de 1 à 4 atomes de carbone;

ou bien

$R_5$ et $R_6$ ou $R_7$ forment éventuellement un cycle de 4 à 7 chaînons qui peut éventuellement contenir comme autres hétéroatomes de l'azote, de l'oxygène ou du soufre, le cycle pouvant être substitué par au moins un groupe alkyle linéaire ou ramifié de 1 à 4 atomes de carbone;

ou bien
$R_6$ ou $R_7$ et $R_8$ forment éventuellement un cycle de 5 à 7 chaînons de formule générale

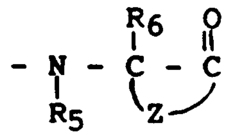

dans laquelle
Z représente un des hétéroatomes d'azote, d'oxygène ou de soufre, le cycle pouvant être substitué par un groupe alkyle linéaire ou ramifié de 1 à 4 atomes de carbone;
X représente le CH, un C-halogène ou l'azote
n est l'un des nombres 0, 1, 2, 3, 4, 5, 6, 7 ou 8,
p est l'un des nombres 1, 2, 3, 4, 5, 6, 7 ou 8 et
m est l'un des nombres 1, 2 ou 3, de préférence 1,
leurs dérivés optiquement actifs ou leurs racémates ainsi qu'éventuellement leurs sels d'addition d'acides physiologiquement acceptables.

**2.** Composés de formule générale I ou II selon la revendication 1, caractérisés en ce que
$R_1$, $R_3$ et A ont la signification donnée ci-dessus,
$R_2$ représente un phényle, le cycle phényle pouvant être substitué, de préférence en position 2, par un halogène, de façon particulièrement préférée par le chlore.
$R_4$ représente une chaîne latérale fonctionnelle de formule générale

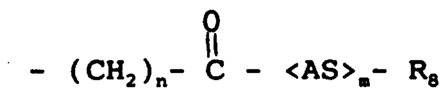

dans laquelle
<AS> représente un aminoacide de formule générale

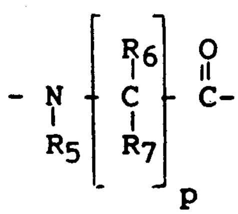

dans laquelle
$R_5$, $R_6$ et $R_7$ sont tels que définis ci-dessus,
$R_8$ a la signification donnée ci-dessus,
X représente le CH ou l'azote,
n est l'un des nombres 0, 1, 2, 3, 4, 5, 6, 7 ou 8,
p est le nombre 1 ou 2 et
m est le nombre 1, 2 ou 3, de préférence 1, leurs dérivés optiquement actifs ou leurs racémates ainsi qu'éventuellement leurs sels d'addition d'acides physiologiquement acceptables.

**3.** Composés de formule générale I ou II selon la revendication 1 ou 2, caractérisés en ce que
$R_1$ représente l'hydrogène, un méthyle, un éthyle, un cyclopropyle, un méthoxy, un éthoxy ou un halogène, de préférence le chlore ou le brome,
$R_2$ représente un groupe phényle, le cycle phényle pouvant être substitué en position 2 par un halogène, de préférence le chlore,
$R_3$ représente l'hydrogène ou un méthyle, de préférence l'hydrogène,
A a la signification donnée ci-dessus,
$R_4$ représente une chaîne latérale fonctionnelle de formule générale

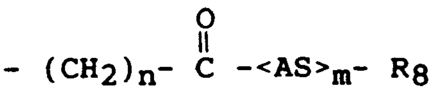

dans laquelle <AS> représente un aminoacide de formule générale

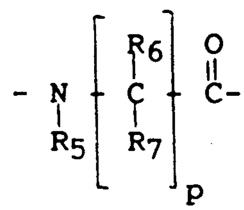

dans laquelle
$R_5$, $R_6$ et $R_7$ sont tels que définis ci-dessus,
$R_8$ a la signification donnée ci-dessus,
X représente un CH ou l'azote,
n, dans le cas où A = a, est l'un des nombres 0 ou 1, et dans le cas où A = b, l'un des nombres 0, 1, 2, 3, 4, 5, 6, 7 ou 8,
p est l'un des nombres 1 ou 2 et
m est l'un des nombres 1 ou 2, de préférence 1,
leurs dérivés optiquement actifs ou leurs racémates ainsi que leurs sels d'addition d'acides physiologiquement acceptables.

**4.** Composés de formule générale I ou II selon l'une des revendications 1 à 3, caractérisés en ce que
$R_1$ représente l'hydrogène, un éthyle, un cyclopropyle, un éthoxy, le chlore ou le brome, de préférence un méthyle ou méthoxy,
$R_2$ représente un phényle, le cycle phényle pouvant être substitué en position 2 par un halogène, de préférence le chlore,
$R_3$ représente l'hydrogène,
A a la signification donnée ci-dessus,
$R_4$ représente une chaîne latérale fonctionnelle de formule générale

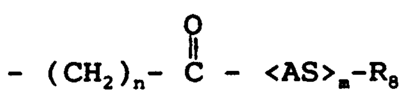

dans laquelle
<AS> représente un α-aminoacide de formule générale

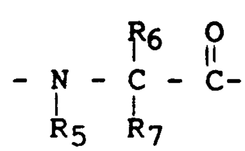

dans laquelle
$R_5$ représente l'hydrogène,
$R_6$ et $R_7$, qui peuvent être identiques ou différents, représentent l'hydrogène, un groupe alkyle linéaire ou ramifié de 1 à 8, de préférence 1 à 5 atomes de carbone, éventuellement substitué par un alcoxycarbonyle, un alkylthio ou alkylsulfonyle ayant 1 à 4 atomes de carbone dans la chaîne alkyle,
$R_8$ représente un alcoxy de 1 à 4 atomes de carbone, de préférence un méthoxy, un éthoxy ou de préférence un groupe amino de formule générale

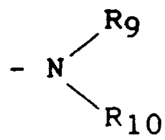

dans laquelle
$R_9$ et $R_{10}$, qui peuvent être identiques ou différents, représentent un groupe alkyle ou alcényle linéaire ou ramifié de 1 à 4 atomes de carbone, ou bien $R_9$ et $R_{10}$ forment un cycle de 5 ou 6 chaînons qui peut contenir comme autres hétéroatomes de l'azote ou de l'oxygène, le cycle pouvant être substitué par au moins un groupe alkyle de 1 à 4 atomes de carbone, de préférence un méthyle,
X représente un CH ou de préférence l'azote,
n,dans le cas où A = a,est l'un des nombres 0 ou 1, de façon particulièrement préférée 0, et dans le cas où A = b, l'un des nombres 0, 1, 2, 3 ou 4, de préférence 0, 1 ou 2,
leurs dérivés optiquement actifs ou leurs racémates ainsi qu'éventuellement leurs sels d'addition d'acides physiologiquement acceptables.

**5.** Composés de formule générale I ou II selon l'une des revendications 1 à 4, caractérisés en ce que $R_4$ représente une chaîne latérale fonctionnelle de formule générale

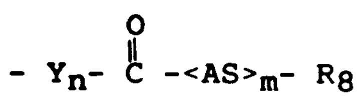

dans laquelle AS représente au moins l'un des aminoacides suivants:Aad, $\gamma$-Abu, $\epsilon$-Aca, Ach, Acp, $\beta$-Aib, $\delta$-Ala, Ama, Apm, Apr, Arg, Asn, Asu, Cys, Gln, Glu, His, Ser, Hyl, Hyp, 3-Hyp, Ise, Lys, Nle, Nva, Pec, Phe, Phg, Pic, Pro, Tle, Pyr, Ser, Thr, Tyr, Trp, Ala, $\beta$-Ala, Gly, Val, Met, Asp, Sar, Met-($O_2$), Aib, Abu, Ile ou Leu,

$R_1$, $R_2$, $R_3$, A, $R_8$, X, n et m ont la signification donnée ci-dessus, leurs dérivés optiquement actifs ou leurs racémates ainsi qu'éventuellement leurs sels d'addition d'acides physiologiquement acceptables.

**6.** Procédé de préparation des composés de formule générale I ou II

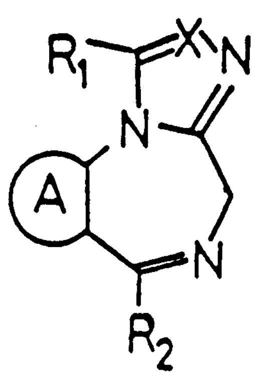

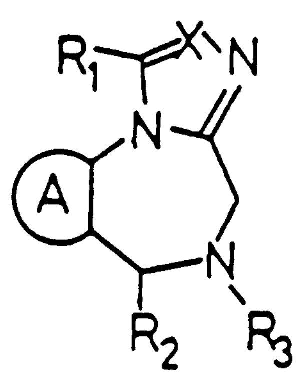

I

II

dans laquelle
$R_1$ représente l'hydrogène, un groupe alkyle linéaire ou ramifié de 1 à 4 atomes de carbone qui peut éventuellement être substitué par un hydroxy ou un halogène, un groupe cyclopropyle, un groupe

alcoxy de 1 à 4 atomes de carbone, un halogène;
$R_2$ représente un phényle, le cycle phényle pouvant être substitué une ou plusieurs fois par un méthyle, un méthoxy, un halogène, un nitro et/ou trifluorométhyle, un α-pyridyle;
$R_3$ représente l'hydrogène ou un groupe alkyle linéaire ou ramifié de 1 à 4 atomes de carbone;
A représente un cycle condensé de formule générale

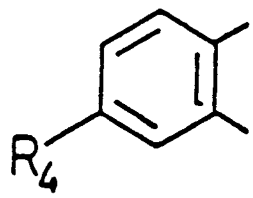

**a**

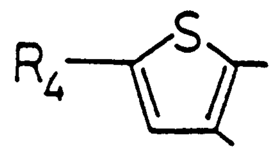

**b**

dans laquelle $R_4$ représente une chaîne latérale fonctionnelle de formule générale

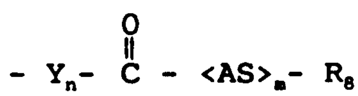

dans laquelle Y représente un groupe alkyle linéaire ou ramifié de n atomes de carbone, <AS> est un aminoacide relié par le N terminal de formule générale

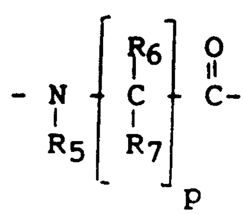

dans laquelle
$R_5$ représente l'hydrogène, un groupe alkyle linéaire ou ramifié de 1 à 5 atomes de carbone;
$R_6$ et $R_7$, qui peuvent être identiques ou différents, représentent l'hydrogène, un groupe alkyle, alcényle ou alcynyle linéaire ou ramifié de 1 à 10 atomes de carbone, un groupe cycloalkyle de 3 à 6 atomes de carbone, un groupe phényle, un groupe benzyle.
$R_6$ et/ou $R_7$, dans le cas d'un groupe alkyle, pouvant éventuellement être substitués une ou plusieurs fois par un hydroxy, un alcoxy en $C_1$-$C_4$, un cycloalkyle de 3 à 7 atomes de carbone, un halogène, un groupe amino qui peut éventuellement être substitué une ou deux fois par un alkyle, un alcoxycarbonyle ou phénylalcoxycarbonyle, linéaires ou ramifiés, et contenant chacun 1 à 4 atomes de carbone dans la chaîne alkyle, un guanidino, un uréido, un carboxy, un alcoxycarbonyle en $C_1$-$C_4$, un cyano, un carboxamide, un alkylcarbonyle en $C_1$-$C_4$, un mercapto, un alkylmercapto en $C_1$-$C_4$, un benzylmercapto, un alkylsulfoxyle en $C_1$-$C_4$, un alkylsulfonyle en $C_1$-$C_4$, un 3-indolyle, un imidazolyle, un pyrazolyle et/ou un amide d'acide de formule générale

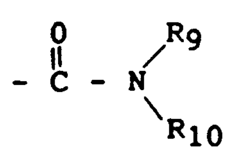

dans laquelle $R_9$ et $R_{10}$, qui peuvent être identiques ou différents, représentent l'hydrogène, un groupe alkyle, alcényle ou alcynyle linéaire ou ramifié de 1 à 4 atomes de carbone, le groupe alkyle pouvant

être substitué par un groupe amino qui peut éventuellement être substitué une ou deux fois par un groupe alkyle linéaire ou ramifié de 1 à 4 atomes de carbone dans la chaîne alkyle, ou par un groupe hydroxy;

ou bien $R_9$ et $R_{10}$ forment éventuellement un cycle de 3 à 6 chaînons qui peut contenir comme autres hétéroatomes de l'azote, de l'oxygène ou du soufre, le cycle pouvant être substitué par au moins un groupe alkyle linéaire ou ramifié de 1 à 4 atomes de carbone;

$R_6$ et/ou $R_7$, quand ils représentent un groupe phényle ou benzyle, peuvent être substitués par au moins un groupe hydroxy, un halogène, un amino ou un groupe alkylamino, dialkylamino, ou alcoxy linéaire ou ramifié de 1 à 4 atomes de carbone;

$R_8$ représente un groupe alcoxy ou alkylthio linéaire ou ramifié de 1 à 4 atomes de carbone éventuellement substitué par un groupe amino de formule générale

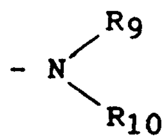

dans laquelle $R_9$ et $R_{10}$ ont la signification donnée ci-dessus, ou un groupe amino de formule générale

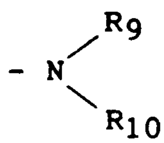

dans laquelle $R_9$ et $R_{10}$ ont la signification donnée ci-dessus;

$R_6$ et $R_7$ forment éventuellement un cycle de 3 à 7 chaînons qui peut contenir comme autres hétéroatomes de l'azote, de l'oxygène ou du soufre et qui peut éventuellement être substitué par au moins un groupe alkyle linéaire ou ramifié de 1 à 4 atomes de carbone;

ou bien

$R_5$ et $R_6$ ou $R_7$ forment éventuellement un cycle de 4 à 7 chaînons qui peut éventuellement contenir comme autres hétéroatomes de l'azote, de l'oxygène ou du soufre, le cycle pouvant être substitué par au moins un groupe alkyle linéaire ou ramifié de 1 à 4 atomes de carbone;

ou bien

$R_6$ ou $R_7$ et $R_8$ forment éventuellement un cycle de 5 à 7 chaînons de formule

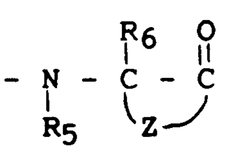

dans laquelle

Z représente un des hétéroatomes d'azote, d'oxygène ou de soufre, le cycle pouvant être substitué par un groupe alkyle linéaire ou ramifié de 1 à 4 atomes de carbone;

X représente un CH, un C-halogène ou l'azote,

n est l'un des nombres 0, 1, 2, 3, 4, 5, 6, 7 ou 8,

p est l'un des nombres 1, 2, 3, 4, 5, 6, 7 ou 8,

m est l'un des nombres 1, 2 ou 3, de préférence 1,

leurs dérivés optiquement actifs ou leurs racémates, caractérisé en ce qu'on fait réagir un composé de formule générale IIIa ou IIIb

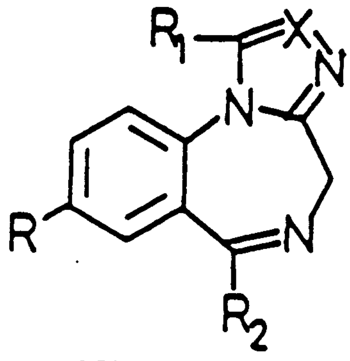

IIIa

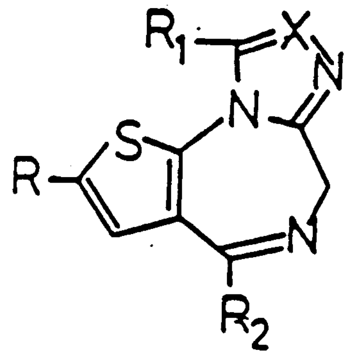

IIIb

dans laquelle les restes $R_1$, $R_2$ et X ont la signification donnée ci-dessus et R est une chaîne latérale de formule générale

$- Y_n -$ COOH

dans laquelle Y et n sont tels que définis ci-dessus,

a) avec un composé de formule générale IV

H - <AS>$_m$- $R_8$

dans laquelle AS, $R_8$ et m ont la signification donnée ci-dessus, en présence d'un carbodiimide ou

b) on transforme l'acide de formule générale IIIa ou IIIb en un dérivé d'acide réactif et on le fait réagir ensuite avec un composé de formule générale IV,

on transforme ensuite éventuellement un composé de formule générale I par réduction en un composé de formule générale II et on transforme éventuellement un composé de formule générale I ou II en ses sels d'addition d'acides.

**7.** Procédé de préparation de composés de formule générale I selon la revendication 6, où $R_1$, $R_2$, $R_4$ et X sont tels que définis ci-dessus et $R_8$ est un groupe amino de formule générale

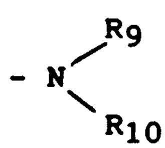

dans laquelle $R_9$ et $R_{10}$ sont tels que définis ci-dessus, caractérisé en ce qu'on fait réagir par des procédés connus en soi un composé de formule générale

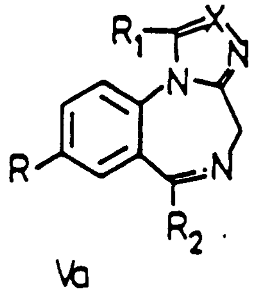

Va

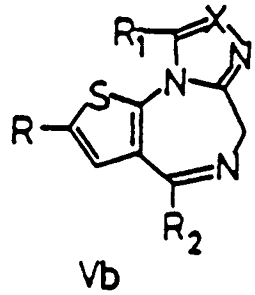

Vb

dans laquelle R signifie -$Y_n$-CO-<AS>$_m$-OH
avec une amine de formule générale

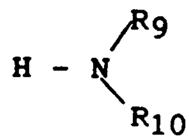

dans laquelle $R_9$ et $R_{10}$ sont tels que définis ci-dessus.

**8.** Compositions pharmaceutiques, contenant comme substances actives des composés de formule générale I ou II en combinaison avec des produits auxiliaires et/ou des supports classiques.

**9.** Procédé de préparation de compositions pharmaceutiques selon la revendication 8, caractérisé en ce qu'on traite des composés de formule générale I ou II avec des produits auxiliaires et/ou des supports galéniques classiques pour donner des formes d'application pharmaceutiques classiques.

**10.** Composés de formule générale I ou II selon l'une des revendications 1 à 5 pour l'utilisation dans la préparation de médicaments à activité antagoniste du PAF.

**11.** Utilisation d'un composé de formule générale I ou II pour la préparation d'un médicament pour le traitement d'états pathologiques et de maladies dans lesquels est impliqué le PAF (facteur d'activation des plaquettes).

**12.** Composés intermédiaires de formule générale

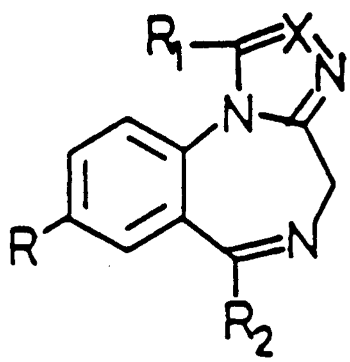

a

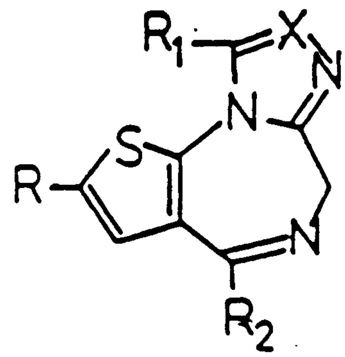

b

III / VI

dans laquelle R représente une chaîne latérale de formule générale

$- Y_n- COOR'$

dans laquelle R' représente l'hydrogène ou un groupe alkyle linéaire ou ramifié de 1 à 4 atomes de carbone et $R_1$, $R_2$, X, Y et n peuvent avoir la signification donnée dans les revendications 1 à 5.

**13.** Composés intermédiaires de formule générale

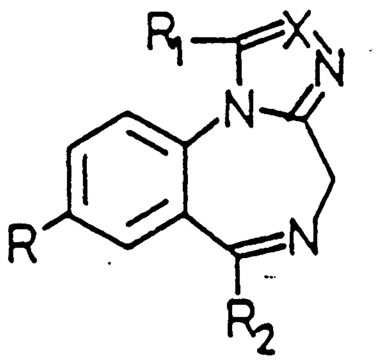

a

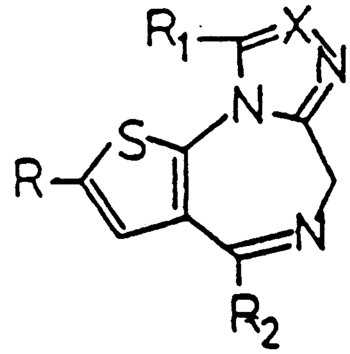

b

V

dans laquelle R est une chaîne latérale de formule générale

- $Y_n$- CO - <AS>$_m$- OH

et $R_1$, $R_2$, X, Y, n, m et <AS> peuvent avoir la signification donnée dans les revendications 1 à 5.